(19) Europäisches Patentamt
European Patent Office
Office européen des brevets

(11) Publication number : **0 532 423 A1**

# (12) EUROPEAN PATENT APPLICATION

(21) Application number : **92402487.0**

(22) Date of filing : **11.09.92**

(51) Int. Cl.$^5$ : **C07F 9/6561,** A61K 31/675, C07F 9/6512

(30) Priority : **12.09.91 EP 91402427**

(43) Date of publication of application : **17.03.93 Bulletin 93/11**

(84) Designated Contracting States : **AT BE CH DE DK ES FR GB GR IE IT LI LU MC NL PT SE**

(71) Applicant : **MERRELL DOW PHARMACEUTICALS INC.**
**2110 East Galbraith Road**
**Cincinnati Ohio 45215-6300 (US)**

(72) Inventor : **Halazy, Serge**
**1 Place des Barrys**
**F-81090 Lagarrigue (FR)**
Inventor : **Danzin, Charles**
**32 Rue Herder**
**F-67000 Strasbourg (FR)**
Inventor : **Nave, Jean-François**
**4 Rue de la Tanche**
**F-67000 Strasbourg (FR)**
Inventor : **Casara, Patrick**
**12 Rue de la Scierie**
**F-67117 Ittenheim (FR)**

(74) Representative : **Gillard, Marie-Louise et al**
**Cabinet Beau de Loménie 55, Rue d'Amsterdam**
**F-75008 Paris (FR)**

## (54) Novel unsaturated acyclic phosphonate derivatives of purine and pyrimidine.

(57) Novel unsaturated acyclic phosphonate derivatives of certain purine or pyrimidines useful as antiviral agents, to methods and intermediates useful for their preparation and to their end-use application as antiviral agents effective against DNA viruses, retroviruses and viruses involved in tumor formation.

EP 0 532 423 A1

Jouve, 18, rue Saint-Denis, 75001 PARIS

This invention relates to novel unsaturated acyclic phosphonate derivatives of certain purine or pyrimidines useful as anti-viral agents, to methods and intermediates useful for their preparation and to their end-use application as antiviral agents effective against DNA viruses (herpes viruses 1 and 2, cytomegalovirus, varicellazoster virus, Epstein-Barr virus), retroviruses (human immunodeficiency viruses 1 and 2 and visna virus) and against viruses involved in tumor formation.

SUMMARY OF THE PRESENT INVENTION

More specifically this invention relates to novel compounds of the formula

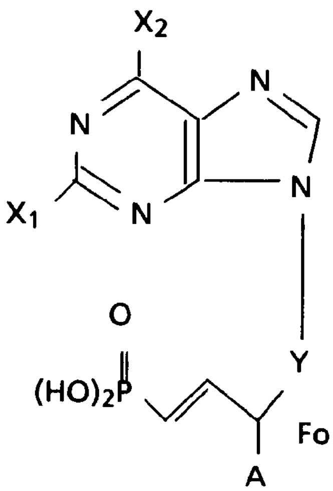

Formula I

or

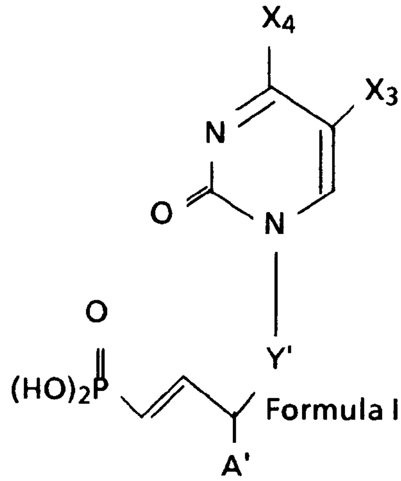

Formula II

the stereoisomeric, tautomeric forms and the pharmaceutically acceptable salts thereof, wherein

Y is $-CH_2-CH_2-$, $-O-CH_2-$ or $-CH_2-O-$;
Y′ is $(CH_2)_n$, $-O-CH_2-$, or $-CH_2-O-$;
n is 1 or 2;
A and A′ are independently H, $-CH_2OH$ or OH;
$X_1$ is H or $NH_2$;
$X_2$ is OH or $NH_2$;
$X_3$ is H or $CH_3$; and
$X_4$ is $NH_2$ or OH;

provided that when Y is $-O-CH_2-$ and A is H, then $X_1$ is not $NH_2$ and $X_2$ is not OH, simultaneously;
provided that when Y is $-CH_2-O-$, then A is OH;
provided that when A is OH, Y is not $-OCH_2-$; when A′ is OH, Y′ is not $-OCH_2-$; and
provided that when Y is $-CH_2-CH_2-$ and A is H, then $X_1$ is not $NH_2$ and $X_2$ is not OH, simultaneously.

The object of the present invention is to provide compounds for the treatment of viral diseases by providing novel compounds which can be administered with a pharmaceutically acceptable carrier to a patient in need of thereof.

PREFERRED EMBODIMENTS OF THE PRESENT INVENTION

The compounds of the present invention are purine derivatives (Formula I) or pyrimidine derivatives (Formula II), herein referred to as bases (dotted line representing the attachment to the rest of the molecule):

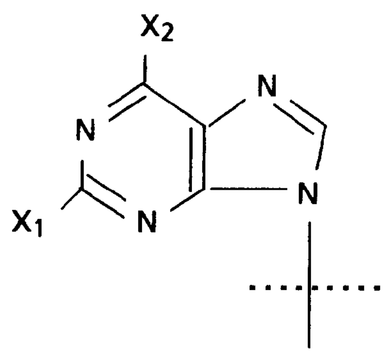

or

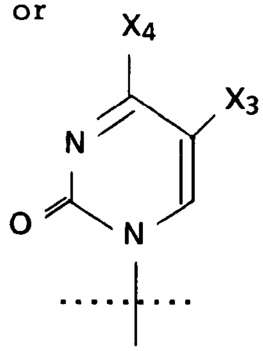

having an alkenyl phosphonate moiety. Linking the alkenyl phosphonate moiety to the purine or pyrimidine derivative is respectively a Y and a Y′ group. The Y group represents an ethylene group, $-CH_2O-$, or $-OCH_2-$. An example of the attachment of Y to the rest of the molecule follows where Y is $-OCH_2-$:

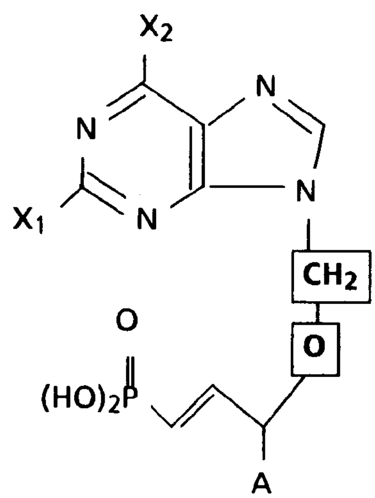

When the Y group is ethylene ($-CH_2-CH_2-$) and A is H, then the purine derivative to which it is attached is not guanine ($X_1$ is not $NH_2$ and $X_2$ is not OH, simultaneously). Also, when Y is $-O-CH_2-$ and A is H, then the purine derivative to which it is attached is not guanine ($X_1$ is not $NH_2$ and $X_2$ is not OH, simultaneously). Additionally, when Y is $-CH_2O-$, then A is OH. When A is OH, Y is not $-OCH_2-$; likewise, when A′ is OH, Y′ is not $-OCH_2-$. The Y′ group of the pyrimidine derivatives represents a methylene or ethylene group [$(CH_2)_n$ wherein n is 1 or 2], $-OCH_2$ group or a $-CH_2O-$ group, the oxygen of which is directly attached to the nitrogen atom of the pyrimidine derivative. Preferably Y is ethylene and/ Y′ is methylene or ethylene.

The A and A′ represent, each independent of the other, H, $-CH_2OH$ or OH. Preferably, A and A′ are each H or $-CH_2OH$.

Preferred pyrimidine type bases are cytidine, uracil, and thymine. Preferred purine bases are 2,6-diaminopurine (DAP), guanine and adenine. Preferably, when the base is guanine, A is not $CH_2OH$.

The term "pharmaceutically acceptable salts" means both acid addition salts and metal and amine salts which are known to be non-toxic and useful derivatives in the preparation of pharmaceutical formulations suitable for end-use applications.

Pharmaceutically acceptable acid addition salts include the known non-toxic organic or inorganic acid addition salts of the base compounds of Formula I and Formula II. Illustrative organic acids which form suitable salts include the mono-, di-, and tricarboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicylic, and 2-phenoxybenzoic acids. Other organic acids which form suitable salts are the sulfonic acids such as methane sulfonic acid and 2-hydroxyethane sulfonic acid. Either the mono- or the di-acid salts can be formed, and such salts can exist in either a hydrated or a substantially anhydrous form. The acid salts are prepared by standard techniques such as by dissolving the free base in an aqueous or aqueous-alcohol solution or other suitable solvent containing the appropriate acid and isolating by evaporating the solution, or by reacting the free base in an organic solvent in which case the salt separates directly or can be obtained by concentration of the solution. In general the acid addition salts of the compounds of this invention are crystalline materials which are soluble in water and various hydrophilic forms, demonstrate higher melting points and an increased stability.

Pharmaceutically acceptable metal and amine salts are those salts which are stable under ambient conditions, and wherein the cation does not contribute significantly to the biological activity of the salt. Suitable metal salts include the sodium, potassium, calcium, barium, zinc, and aluminium salts. The sodium and potassium salts are preferred. Suitable amine salts are prepared from amines which have sufficient basicity to form a stable salt, and preferably include those amines which are frequently used in medicinal chemistry because of their low toxicity and acceptability for medical use. These include the trialkylamines such as triethylamine, and others including procaine, dibenzylamine, N-benzyl-betaphenethylamine, ephenamine, and N,N'-dibenzylethylenediamine, dehydroabietylamine, N-ethylpiperidine, benzylamine, and dicyclohexylamine.

"Stereoisomeric forms" of the compounds of Formula I and Formula II is a general term for all isomers of these compounds that differ only in the orientation of their atoms in space which include mirror image isomers (enantiomers), geometric (cis/trans) isomers, and isomers of drugs with more than one chiral center that are not mirror images of one another (diastereoisomers). Mixtures may be resolved or isolated according to conventional and standard procedures well known in the art, e.g., chromatographic separation, fractional crystallization, use of optically active acids, enzymatic resolution and the like. Tautomeric enol-keto forms may exist at the 6-position of the purine nucleus, and the pyrimidine will exhibit amine-imine tautomeric forms.

The compounds of this invention may be prepared by the application of analogous chemical reactions known in the art, using reactants which are already known or which may be prepared using standard processes and techniques known in the art. In its essence, the general method for preparing the compounds of Formula I and Formula II may be depicted by the following reaction scheme I.

## Scheme I

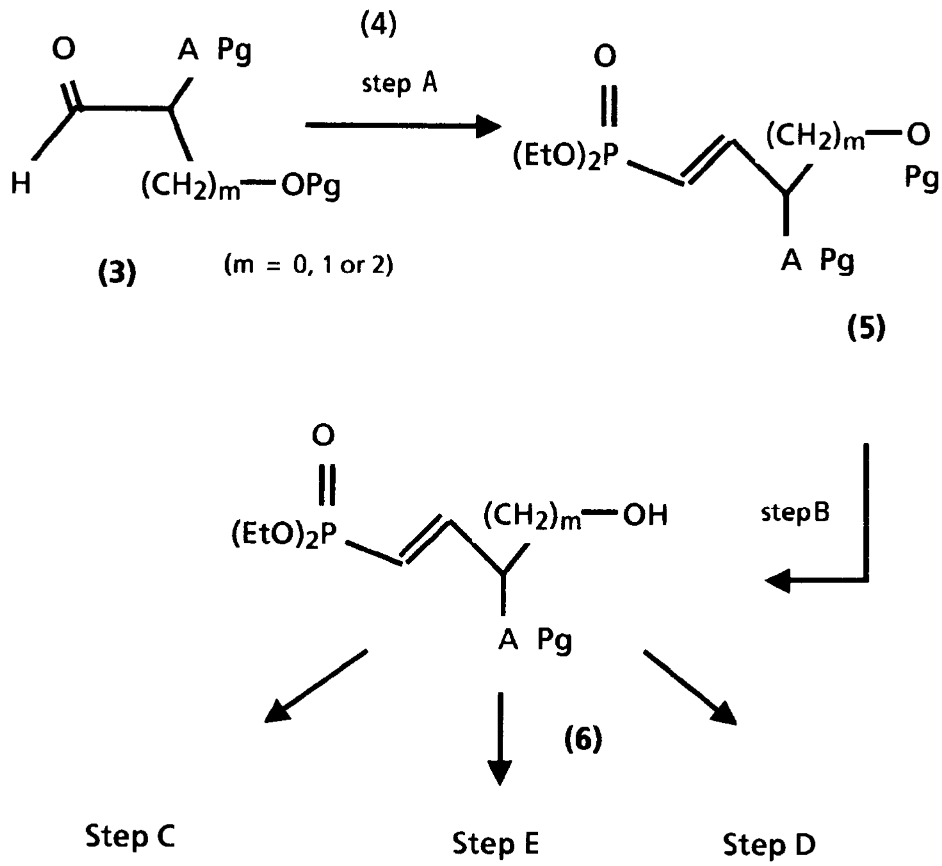


(3)

(5)

(6)

**Scheme I (continued)**

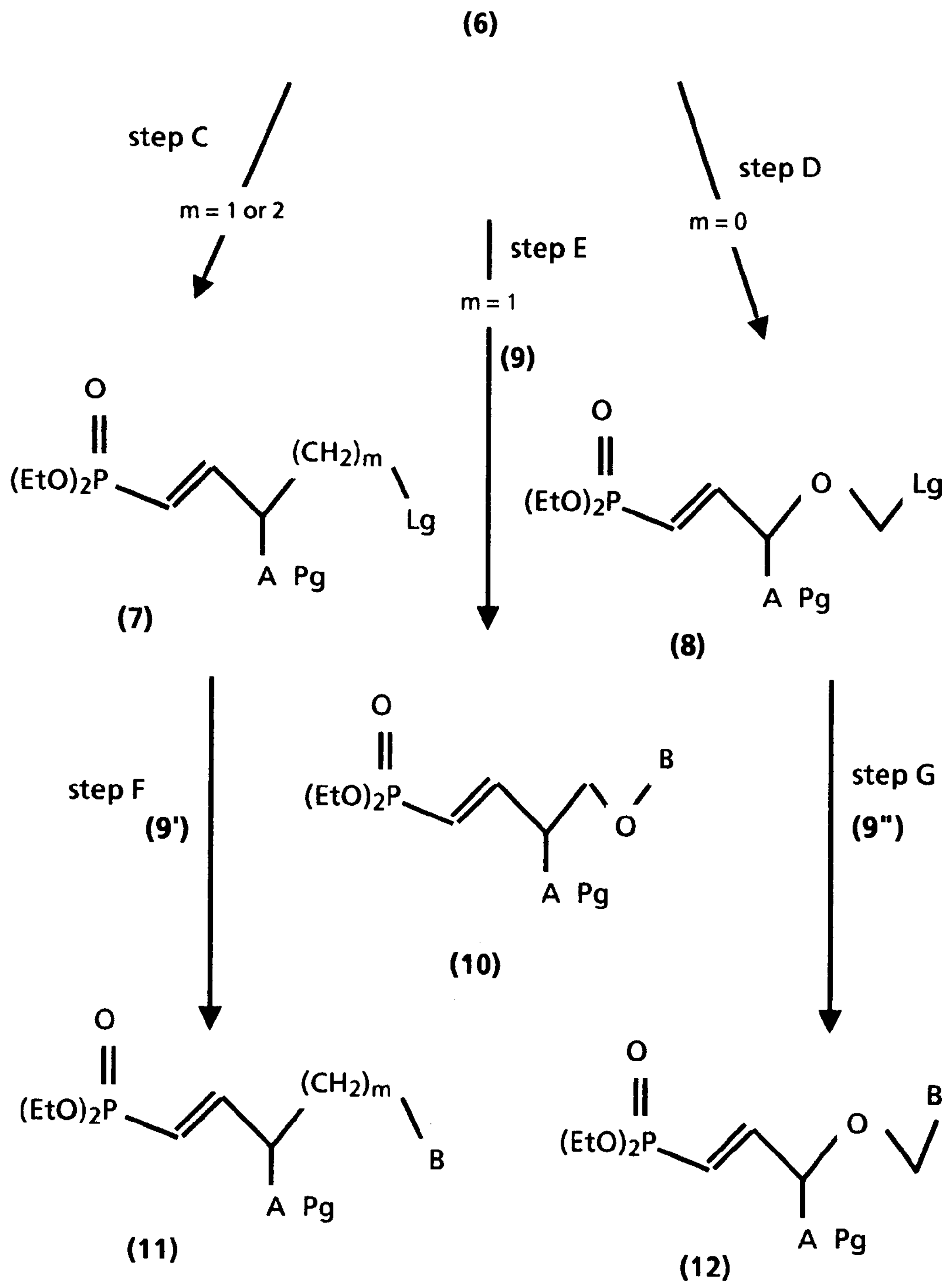


As used herein "Pg" means an appropriate protecting group. Appropriate protecting groups can be found in *Protective groups in Organic Synthesis, 2nd ed.,* Theodora W. Greene, John Wiley & Sons, Inc., New York 1991, and include known esters and ethers with acetyl, benzoyl, and tetrahydropyranyl. When desirable, selective hydroxy protecting groups such as t-butyldimethylsilyl ether or t-butyldiphenylsilyl ether may be used when it is necessary to remove selectively one protecting group. Obviously, when A is H, a protecting group is not necessary. The $(CH_2)_m$ moiety represents methylene, ethylene or is not present, i.e, respectively where m is 1, 2 or 0. "Et" means ethyl but other suitable radicals which protect the phosphonic acid moiety may be used, e.g., $C_{1-8}$ alkyls, particularly isopropyl, phenyl, benzyl, or phenylalkyl. "Lg" means a leaving group. Suitable leaving groups include triflates, alkyl or aryl sulfonates (e.g., $CF_3SO_2O$, tosylate, mesylate) and halides (Br, Cl, or I). In Scheme I, the moiety "A" represents A and A′ as used in formulas I and II. In Scheme I, note that when m is 0 then A is not OH. The purine and pyrimidine bases as defined in Formulas I and II are designated as "B".

Typically the preparations of the compounds produced by Scheme I use reactions of an activated nucleophile with an electrophile bearing a suitable leaving group usually in a suitable anydrous solvent such as tetrahydrofuran, dimethylformamide, ether or the like, generally in an inert atomosphere of nitrogen or argon at temperatures ranging from about -78°C to about room temperature, depending upon the specific reactive moieties involved. Once the desired reactions occurs, the necessary modifications can be made such as further deprotection of moieties, formation of appropriate leaving groups, de-esterification of the phosphonate esters, etc.

Referring to Scheme I, the aldehyde compound (3) is starting material which is readily available either through commercial sources or the synthesis is described herein or described in available printed publications. In step A, compound (3) is reacted with the appropriate compound (4) to give the corresponding phosphorylated alkenylalcohol compound (5).

For example, the appropriate aldehyde compound (3) is contacted with a slight excess of one molar equivalent of the lithium salt of tetraethylenebisphosphonate in the presence of a solvent such as tetrahydrofuran. The reactants are typically stirred together for a period of time ranging from 10-48 hours and at a temperature range of about -78°C. The corresponding phosphorylated alkenylalcohol compound (5) is recovered from the reaction zone by extractive methods well known in the art.

In step B, the terminal hydroxy group of the phosphorylated alkenylalcohol compound (5) is selectively deprotected to produce the corresponding alcohol (6). The protecting group is cleaved by standard methods depending upon the type of protecting group used. A preferred protecting group for the hydroxy group is tert-butyldimethylsilyl which may be cleaved by known methods. From the alcohol (6) either the base or leaving groups are added.

In step C wherein m is 1 or 2, the alcohol (6) is treated to remove the terminal hydroxy group and add a methylene with a suitable leaving group to provide the phosphorylated alkenyl (7). For example, the appropriate phosphorylated alkenylalcohol (6) is reacted with a slight excess of one molar equivalent of mesyl chloride at about -10°C under argon in the presence of a non-nucleophilic base such as triethylamine and dimethylamino pyridine in the presence of an organic solvent such as anhydrous dichloromethane. The reactants are typically stirred together for a period of time ranging from 10-48 hours at about 0°C to room temperature. The so-produced mesyloxy alkenyl phosphonic acid, dialkyl ester is treated to remove the mesyloxy group and replace it with an iodine atom. This may be accomplished by reacting the so-produced mesyloxy alkenyl phosphonic acid, dialkyl ester with a halogen leaving group such as sodium iodide in the presence of a solvent such as acetone and heated to about 55°C for 10-48 hours. The corresponding phosphorylated alkyl (7) is recovered from the reaction zone by extractive methods well known in the art.

In step D wherein m is 0, the terminal oxygen is maintained and a methylene group with a suitable leaving group is attached thereto to form the phosphorylated alkenylether (8). For example, the appropriate phosphorylated alkenylalcohol (6) is reacted with a slight excess of one molar equivalent of paraformaldehyde with anhydrous hydrochloric acid bubbled into the mixture in the presence of an organic solvent such as dichloromethane at about 0°C for about 15 minutes. The reactants are typically stirred together for a period of time ranging from 10-48 hours and at a temperature range of from 0°C to room temperature to give the chloromethoxy alkyenylphosphonic acid dialkylester (8) which is recovered from the reaction zone by extractive methods well known in the art.

In steps E, F, and G, to the respective corresponding compounds (6), (7) and (8) are added the appropriate purine or pyrimidine base (9) as defined herein to give respectively compounds 10, 11 and 12. Adding the bases (9), (9′) and (9″) to the respective corresponding compounds (6), (7) and (8) may require certain protecting groups and specific conditions for the particular bases which are well known in the art and further exemplified hereafter. Further steps may be required to deprotect the A moiety and the base moieties which are well known in the art. The bases described here are treated in order to effect the reaction with the hydroxy group of alcohol (6) and the leaving groups of compounds (7) and (8), e.g., Base (9) is 1-hydroxypyrimidine or 9-hydroxypurine (sometimes with the appropriate functional groups of base protected) to effect the Mitsunobu reaction, Base (9′) is the salt form of the base such as sodium or potassium to effect the displacement of the leaving group; and base (9″) is the silylated derivative of the base.

For example, in step F the phosphorylated alkyl (7) is reacted with about one molar molar equivalent of the appropriate base such as 5-N-acetylcytosine in the presence of an organic solvent such as anhydrous dimethylformamide and a base such as potassium carbonate. The reactants are stirred for from 10-48 hours at from about 0°C to room temperature. The expected compound is recovered and mixed with a basic solution

such as ammonia in ethanol. It may be desirable to further deprotect the compound by standard means.

For example, in the conditions of the Mitsunobu reaction, in step E the phosphorylated alkenylalcohol (6) is reacted with a base (9) such as 1-hydroxythymine and condensating agents such as triphenylphosphine and diethylazodicarboxylate in the presence of an organic solvent such as anhydrous tetrahydrofuran. The reaction mixture is stirred at from 0°C to about room temperature for from 10-48 hours. The expected compound is recovered by conventional means. The compound may be further deprotected if needed.

For example, in step G the phosphoryl-alkenylether (8) is reacted with a base (9) prepared by mixing bistrimethylsilylacetamide and adenine in an organic solvent such as anhydrous 1,2-dichloroethane under argon until dissolved. The chloromethyl ether described in step D is added in the presence of an organic solvent such as dilute 1,2-dichloroethane. The reactants are stirred from 10-48 hours at from 0°C to about room temperature under argon. The reaction is hydrolized by, for example, methanol and recovered by standard means. Further deprotection may be needed.

De-esterification of the dialkylphosphoryl moiety may be by various means. For example, a diethylphosphoryl compound may be reacted with trimethylsilylbromide in anhydrous acetonitrile and stirred at from 0°C to room temperature from 10-48 hours under argon. The de-esterified compound can be recovered by conventional means.

The following examples will illustrate some of the methods by which the compounds of this invention may be prepared. These examples are illustrative only and the preparation of the compounds of the present invention should not be limited to the syntheses described herein. For the purposes of this patent application, "dihydroxyphosphoryl" means the same as "phosphonyl" or "phosphono" [$(HO)_2(O)P$-].

## EXAMPLE 1

## E-1-[5-(DIHYDROXYPHOSPHORYL)PENT-4-ENYL]CYTOSINE

STEP A:

4-($^t$BUTYLDIMETHYLSILYLOXY)-1-BUTANOL

$^t$Butyldimethylsilyl chloride (25 g, 165 mmol) dissolved in 60 ml of anhydrous dichloromethane was added to a stirred solution of 13.6 g (150 mmol) of 1,4-butanediol, 25 ml of triethylamine and 2 g of 4-dimethyl aminopyridine in 300 ml of dichloromethane. The reaction mixture was stirred at 20°C for 20 hours, washed with aqueous saturated ammonium chloride, bicarbonate and brine, dried, evaporated and purified by flash chromatography on silica gel to give 18.1 g of the expected product 4-($^t$Butyldimethylsilyloxy)-1-butanol (59% yield).

STEP B:

[5-($^t$BUTYLDIMETHYLSILYLOXY)-1-PENTENYL]PHOSPHONIC ACID, DIETHYL ESTER

4-($^t$butyldimethylsilyloxy)-1-butanol (18.1 g, 89 mmol) dissolved in 25 ml of dichloromethane was added dropwise to a vigorously stirred suspension of pyridinium chlorochromate (20.7 g) in 100 ml of dichloromethane. After 1½ hours, 200 ml of ether were added and the mixture is stirred for 1½ hours. The mixture was filtered over florisil sand,evaporated to give 13 g of crude product which is distilled (48°C/0.35 mmHg) to give 7.4 g of 4-($^t$butyldimethylsilyloxy)-1-butenal (42% yield). This aldehyde was added to the lithium salt of tetraethylmethylenebisphosphonate (prepared by adding 50 mmol of n-butyllithium to a solution of 50 mmol of tetraethylmethylenebisphosphonate in 60 ml of tetrahydrofuran at -78°C). The reaction mixture was stirred at -78°C for 3 hours, at 20°C for 20 hours, quenched with aqueous saturated ammonium chloride and evaporated under reduced pressure. The residue was extracted with ethyl acetate (5 x 60 ml), evaporated and purified by flash chromatography on silica gel to give 11.07 g of the expected product [5-($^t$butyldimethylsilyloxy)-1-pentenyl]phosphonic acid, diethyl ester (91% yield).

STEP C:

(5-MESYLOXY-1-PENTENYL)PHOSPHONIC ACID DIETHYL ESTER

Trihydrated tetrabutylammonium fluoride (5.63 g, 18 mmol) was added to a stirred solution of [5-($^t$butyldimethylsilyloxy)-1-pentenyl]phosphonic acid, diethyl ester (4 g, 12 mmol) in 25 ml of tetrahydrofuran at 20°C. After 2 hours, the reaction mixture was evaporated; the residue was dissolved in ethyl acetate, washed with water and bicarbonate, dried over sodium sulfate, filtered, evaporated and purified by flash chromatography on silica gel to give 2.27 g (86%) of the expected product which was dissolved in anhydrous dichloromethane and treated successively by 1.6 ml of triethylamine, 0.15 g of 4-dimethylamino pyridine and 1.8 g of mesyl chloride at -10°C under argon. The reaction mixture was stirred at 20°C for 20 hours, evaporated and purified by flash chromatography on silica gel to give 2.72 g of the expected product (5-mesyloxy-1-pentenyl)phosphonic acid, diethyl ester (72% yield).

STEP D:

(5-IODO-1-PENTENYL)PHOSPHONIC ACID DIETHYL ESTER

A mixture of (5-mesyloxy-1-pentenyl)phosphonic acid diethyl ester (3 g, 10 mmol) and sodium iodide (1.65 g, 11 mmol) in acetone (20 ml) was heated at 55°C overnight. Then, the mixture was concentrated *in vacuo*, dissolved in ether and washed with brine and purified by flash chromatography on silica gel to give the title product (2.9 g, 88%).

STEP E:

E-1-[5-(DIETHOXYPHOSPHORYL)PENT-4-ENYL] - N-ACETYLCYTOSINE

A mixture of N-acetylcytosine (1.68 g, 11 mmol), (5-iodo-1-pentenyl)phosphonic acid diethyl ester (3.32 g, 10 mmol) and potassium carbonate (1.52 g, 11 mmol) in anhydrous dimethylformamide (20 ml) was stirred at 20°C for 2 days. Then, the reaction mixture was concentrated *in vacuo*, diluted with brine and extracted with dichloromethane. The title product was obtained by flash chromatography on silica gel (2.7 g, 69%).

STEP F:

E-1-[5-(DIETHOXYPHOSPHORYL)PENT-4-ENYL]CYTOSINE

A mixture of E-1-[5-(diethoxyphosphoryl)pent-4-enyl] N-acetylcytosine (1.9 g, 5.3 mmol) in a saturated ethanolic solution of ammonia (50 ml) hermetically closed, was stirred overnight at 20°C. Then, the reaction mixture was concentrated *in vacuo* and the title product was obtained by flash chromatography on silica gel (1.6 g, 89%).

STEP G:

E-1-[5-(DIHYDROXYPHOSPHORYL)PENT-4-ENYL]CYTOSINE

A mixture of E-1-[5-(diethoxyphosphonyl)pent-4-enyl]cytosine (1.39 g, 4.3 mmol) trimethylsilylbromide (3.4 ml, 25 mmol) in anhydrous acetonitrile (20 ml) was stirred at 20°C overnight under argon. Then, the reaction mixture was concentrated *in vacuo*, diluted with absolute ethanol and crystallized on cooling to afford the pure title product (0.6 g, 65%).

**EXAMPLE 2**

**E-1-[5-(DIHYDROXYPHOSPHORYL)PENT-4-ENYL]THYMINE**

STEP A:

E-1-[5-(DIHETHOXYPHOSPHORYL)PENT-4-ENYL]THYMINE

A mixture of thymine (1.38 g, 11 mmol), (5-iodo-1-pentenyl)phosphonic acid diethyl ester (3.32 g, 10 mmol - described in Step D, Example 1), potassium carbonate (1.92g, 11 mmol) in anhydrous dimethylformamide (20 ml) was stirred at 20°C for 3 days under argon. Then, the reaction mixture was concentrated *in vacuo*, diluted with brine and extracted with dichloromethane. The title product was purified by flash chromatography on silica gel (0.6 g, 18%).

STEP B:

E-1-[5-(DIHYDROXYPHOSPHORYL)PENT-4-ENYL]THYMINE

A mixture of E-1-[5-(diethoxyphosphoryl)pent-4-enyl] thymine (0.6 g, 1.8 mmol) and trimethylsilylbromide (1.4 ml, 11 mmol) in anhydrous acetonitrile (10 ml) was stirred at 20°C overnight under argon. Then, the reaction mixture was concentrated *in vacuo*, diluted with absolute ethanol and crystallized on cooling to afford the title product (0.61 g, 90%).

**EXAMPLE 3**

**E-1-[4 -(DIHYDROXYPHOSPHORYL)BUT-3-ENYL]CYTOSINE**

STEP A:

3-($^t$BUTYLDIMETHYLSILYLOXY)-1-PROPANOL
$^t$Butyldimethylsilyl chloride (25 g, 165 mmol) dissolved in 60 ml of anhydrous dichloromethane is added to a stirred solution of 1,3-propanediol (10.5 g, 150 mmol) triethylamine (25 ml) and 4-dimethyl aminopyridine (2 g) in 300 ml of dichloromethane. The reaction mixture is stirred at 20°C for 20 hours, washed with aqueous saturated ammonium chloride, bicarbonate and brine, dried, evaporated and purified by flash chromatography on silica gel to give the expected product 3-($^t$butyldimethylsilyloxy)-1-propanol (16.6 g, 60% yield).

STEP B:

[4-($^t$BUTYLDIMETHYLSILYLOXY)BUT-1-ENYL]PHOSPHONIC ACID, DIETHYL ESTER
3-($^t$Butyldimethylsilyloxy)-1-propanol (16.6 g, 90 mmol) dissolved in dichloromethane (25 ml) is added dropwise to a vigorously stirred suspension of pyridinium chlorochromate (21 g) in dichloromethane (100 ml). After 1½ hours, ether (200 ml) is added and the mixture is stirred for 1½ hours. The mixture is filtered over florisil and evaporated to give the crude product (12 g) which is distilled (45°C/0.30 mmHg) to give 8.0 g of 3-($^t$Butyldimethylsilyloxy)-1-propanal (48% yield). This aldehyde is added to the lithium salt of tetraethylmethylenebisphosphonate (prepared by adding 50 mmol of n-butyllithium to a solution of 50 mmol of tetraethylmethylenebisphosphonate in 60 ml of tetrahydrofuran at -78°C). The reaction mixture is stirred at -78°C for 3 hours, at 20°C for 20 hours, quenched with aqueous saturated ammonium chloride and evaporated under reduced pressure. The residue is extracted with ethyl acetate (5 x 60 ml), evaporated and purified by flash chromatography on silica gel to give the expected product [4-($^t$butyldimethylsilyloxy)but-1-enyl]phosphonic acid, diethyl ester (14.3 , 85% yield).

STEP C:

(4-MESYLOXY-BUT-1-ENYL)PHOSPHONIC ACID, DIETHYL ESTER
Trihydrated tetrabutylammonium fluoride (5.63 g, 18 mmol) is added to a stirred solution of [4-($^t$butyldimethylsilyloxy)but-1-enyl] phosphonic acid, diethyl ester (3.2 g, 10 mmol) in tetrahydrofuran (20 ml) at 20°C. After 2 hours, the reaction mixture is evaporated; the residue is dissolved in ethyl acetate, washed with water and bicarbonate, dried over sodium sulfate, filtered, evaporated and purified by flash chromatography on silica gel to give (1.55 g, 90%) of the expected product which is dissolved in anhydrous dichloromethane and treated successively by 1.6 ml of triethylamine, 0.15 g of 4-dimethylamino pyridine and 1.8 g of mesyl chloride at -10°C under argon. The reaction mixture is stirred at 20°C for 20 hours, evaporated and purified by flash chromatography on silica gel to give the expected product (4-mesyloxy-1-butenyl )phosphonic acid, diethyl ester (2.1 g, 90% yield).

STEP D:

(4-IODO-1-BUTENYL)PHOSPHONIC ACID DIETHYL ESTER
A mixture of (4-mesyloxy-but-1-enyl)phosphonic acid, diethyl ester (2.50 g, 10 mmol) and sodium iodide (1.65 g, 11 mmol) in acetone (20 ml) is heated at 55°C overnight. Then, the mixture is concentrated *in vacuo*, dissolved in ether and washed with brine and purified by flash chromatography on silica gel to give the title product (2.4 g, 85%).

STEP E:

E-1-[4-(DIETHOXYPHOSPHORYL)BUT-3-ENYL]-N-ACETYLCYTOSINE
A mixture of N-acetylcytosine (3.40 g, 22 mmol) (4-iodo-1-butenyl)phosphonic acid diethyl ester (6.5 g, 20 mmol) and potassium carbonate (3.1 g, 22 mmol) in anhydrous dimethylformamide (30 ml) is stirred at 20°C for 2 days. Then, the reaction mixture is concentrated *in vacuo*, diluted with brine and extracted with dichloromethane. The title product is obtained by flash chromatography on silica gel (4.8 g, 69%).

STEP F:

E-1-[4-(DIETHOXYPHOSPHORYL)BUT-3-ENYL]CYTOSINE

A mixture of E-1-[4-(diethoxyphosphonyl)but-3-enyl]-N-acetylcytosine (3.2 g, 10 mmol) in a saturated ethanolic solution of ammonia (100 ml), hermetically closed, is stirred overnight at 20°C. Then, the reaction mixture is concentrated *in vacuo* and the title product is obtained by flash chromatography on silica gel (2.75 g, 90%).

STEP G:

E-1-[4-(DIHYDROXYPHOSPHORYL)BUT-3-ENYL]CYTOSINE

A mixture of E-1-[4-(diethoxyphosphoryl)but-3-enyl] cytosine (1.55 g, 5 mmol), trimethylsilylbromide (4.0 ml, 30 mmol) in anhydrous acetonitrile (30 ml) is stirred at 20°C overnight under argon. Then, the reaction mixture is concentrated *in vacuo*, diluted with absolute ethanol and crystallized on cooling to give the title product (1.05 g, 70%).

**EXAMPLE 4**

**E-1-[4-(DIHYDROXYPHOSPHORYL)BUT-3-ENYL]THYMINE**

STEP A:

E-1-[4-(DIETHOXYPHOSPHORYL)BUT-3-ENYL]THYMINE

A mixture of thymine (1.38 g, 11 mmol), (4-iodo-1-butenyl)phosphonic acid diethyl ester (3.20 g, 10 mmol), potassium carbonate (1.52 g, 11 mmol) in anhydrous dimethylformamide (20 ml) is stirred at 20°C for 3 days under argon. Then, the reaction mixture is concentrated *in vacuo*, diluted with brine and extracted with dichloromethane. The title product is purified by flash chromatography on silica gel (0.75 g, 25%).

STEP B:

E-1-[4-(DIHYDROXYPHOSPHORYL)PROP-2-ENYLOXY]METHYL CYTOSINE

A mixture of E-1-[4-(diethoxyphosphoryl)but-3-enyl]thymine (0.7 g, 2.3 mmol) and trimethylsilylbromide (1.7 ml, 13 mmol) in anhydrous acetonitrile (10 ml) is stirred at 20°C overnight under argon. Then, the reaction mixture is concentrated in vacuo, diluted with absolute ethanol and the title product crystallized on cooling (0.60 g, 88%).

**EXAMPLE 5**

**E-1-[(3-DIHYDROXYPHOSPHORYL-PROP-2-ENYLOXY)METHYL]CYTOSINE**

STEP A:

2-TETRAHYDROPYRANYLOXYACETALDEHYDE

A mixture of 2-tetrahydropyranyloxy-1-ethanol (14.6 g, 100 mmol), N-methylmorpholine-N-oxyde (NMMNO), (20.25 g, 150 mmol) and tetrapropylammonium perruthenate (TPAP) (52 g, 150 mmol), molecular sieves in powder in anhydrous dichloromethane was stirred 2 hours at 20°C. Then, the reaction mixture was filtered off through a pad of celite, concentrated and the crude title product (10.8 g, 50%) was used for the next step without further purification.

STEP B:

(3-TETRAHYDROPYRANYLOXY-1-PROPENYL)PHOSPHONIC ACID DIETHYLESTER

A solution of n-butyllithium (25 ml, 2M in hexane, 50 mmol) was added to a solution of tetraethylmethylenebisphosphonate (14.4 g, 50 mmol in anhydrous tetrahydrofuran (50 ml) at -78°C under argon. After 15 minutes, a solution of 2-tetrahydropyranyloxy-acetaldehyde (7.2 g, 50 mmol) in anhydrous tetrahydrofuran (20 ml) was added dropwise at -78°C. The reaction mixture is stirred 3 hours at -78°C, then, overnight at 20°C. Then, the reaction mixture was quenched with a saturated aqueous solution of ammonium chloride and extracted with diethylether. The title product is obtained by flash chromatography on silica gel (5.6 g, 40%).

STEP C:

(3-HYDROXY-1-PROPENYL)PHOSPHONIC ACID DIETHYLESTER

A mixture of (3-tetrahydropyranyloxy-1-propenyl)phosphonic acid diethyl ester (2.8 g, 10 mmol) and p-toluenesulfonic acid (0.6 g) in absolute ethanol (10 ml) was stirred overnight at 20°C. The reaction mixture was concentrated *in vacuo* and the title product purified by flash chromatography on silica gel (1.37 g, 71%).

STEP D:

[3-(CHLOROMETHOXY)-1-PROPENYL]PHOSPHONIC ACID DIETHYLESTER

Anhydrous hydrochloric acid was bubbled into a mixture of paraformaldehyde (0.17 g, 5 mmol), 3-hydroxy-1-propenylphosphonic acid diethylester (0.95 g, 5 mmol) in anhydrous 1,2-dichloroethane (5 ml) at 0°C for 15 minutes. Then, the reaction mixture was stirred at 20°C overnight, concentrated *in vacuo*. The chloromethyl ether formation was controlled by NMR analysis, and was used in the next step without further purification.

STEP E:

E-1-[3-(DIETHOXYPHOSPHORYL)PROP-2-ENYLOXY]METHYL ACETYLCYTOSINE

A mixture of bistrimethylsilylacetamide (2.5 ml) N-acetylcytosine (0.75 g, 5 mmol) in anhydrous 1,2-dichloroethane (10 ml) was heated overnight under argon. Then, the residue obtained in Step D diluted in 1,2-dichloroethane (5 ml) and tetrabutyl ammonium iodide (1.85 g, 5 mmol) were added and the resulting mixture was stirred overnight at 20°C under argon. Then, the reaction mixture was hydrolized with methanol (5 ml), concentrated *in vacuo*, diluted with brine and extracted with dichloromethane. The title product was purified by flash chromatography on silica gel (0.95 g, 47%).

STEP F:

E-1-[3-(DIETHOXYPHOSPHORYL)PROP-2-ENYLOXY]METHYLCYTOSINE

A mixture of E-1-[3-(diethoxyphosphoryl)prop-2-enyloxy]methyl acetylcytosine (0.72 g, 2 mmol) in a saturated ethanolic solution of ammonia (20 ml), hermetically closed, was stirred overnight at 20°C. Then, the mixture was concentrated *in vacuo* and the title product was purified by flash chromatography on silica gel (0.54 g, 85%).

STEP G:

E-1-[3-(DIHYDROXYPHOSPHORYL)PROP-2-ENYLOXY]METHYLCYTOSINE

A mixture of E-1-[3-(diethoxyphosphoryl)prop-2-enyloxy]methylcytosine (0.32 g, 1 mmol) and trimethylsilyl bromide (0.8 ml, 6 mmol) in anhydrous acetonitrile (5 ml) was stirred at 20°C overnight under argon. Then, the reaction mixture was concentrated *in vacuo*, diluted with absolute ethanol and crystallized on cooling to afford the title product (0.2 g, 70%).

**EXAMPLE 6**

**E-1-[(3-DIHYDROXYPHOSPHORYL-2-PROPENYLOXY)METHYL]THYMINE**

STEP A:

E-1-[(3-DIETHOXYPHOSPHORYL-2-PROPENYLOXY)METHYL]THYMINE

A mixture of bistrimethylsilylacetamide (2.5 ml), thymine (0.625 g, 5 mmol) in anhydrous 1,2-dichloroethane (5 ml) is heated overnight under argon. Then, the chloromethyl ether (obtained in Step D, Example 5) diluted in 1,2- dichloroethane (5 ml) and tetrabutylammonium iodide (1.85 g, 5 mmol) are added and the resulting mixture is stirred overnight at 20°C under argon. Then, the reaction mixture is hydrolized with methanol (5 ml) concentrated *in vacuo*, diluted with brine and extracted with dichloromethane. The product is obtained by flash chromatography on silica gel (0.85 g, 52%).

STEP B:

E-1-[(3-DIHYDROXYPHOSPHORYL-2-PROPENYLOXY)METHYL] THYMINE

A mixture of E-1-[(3-diethoxyphosphoryl-2-propenyloxy)methyl]thymine (0.65 g, 2 mmol) and trimethylsilylbromide (1.6 ml, 12 mmol) in anhydrous acetonitrile (10 ml) is stirred at 20°C overnight under argon. Then, the reaction mixture is concentrated *in vacuo*, diluted with absolute ethanol and crystallized on cooling to give the title product (0.42 g, 75%).

**EXAMPLE 7**

**E-9-[(3-DIHYDROXYPHOSPHORYL-2-PROPENYLOXY)METHYL]ADENINE**

STEP A:

E-9-[(3-DIETHOXYPHOSPHORYL-2-PROPENYLOXY)METHYL]ADENINE

A mixture of bistrimethylsilylacetamide (2,5 ml), adenine (0.67 g, 5 mmol) in anhydrous 1,2- dichloroethane (5 ml) is heated under argon until complete dissolution. Then, the chloromethyl ether (obtained in Step D, Example 5) diluted in 1,2- dichloroethane (5 ml) is added and the resulting mixture is stirred overnight at 20°C under argon. Then, the reaction mixture is hydrolized with methanol (5 ml) concentrated *in vacuo*, diluted with brine and extracted with dichloromethane. The product is isolated by flash chromatography on silica gel (0.95 g, 55%).

STEP B:

E-9-[(3-DIHYDROXYPHOSPHORYL-2-PROPENYLOXY)METHYL]ADENINE

A mixture of E-9-[(3-diethoxyphosphoryl-2-propenyloxy)methyl]adenine (0.68 g, 2 mmol) and trimethylsilylbromide (1.6 ml, 12 mmol) in anhydrous acetonitrile (10 ml) is stirred at 20°C overnight under argon. Then, the reaction mixture is concentrated *in vacuo*, diluted with absolute ethanol and crystallized on cooling to afford the title product (0.39 g, 65%).

**EXAMPLE 8**

**E-1-[4-(DIHYDROXYPHOSPHORYL)BUT-3-ENYLOXY]CYTOSINE**

STEP A:

(4-HYDROXY-1-BUTENYL)PHOSPHONIC ACID DIETHYLESTER

Trihydrated tetrabutylammonium fluoride (5.63 g, 18 mmol) is added to a stirred solution of 4-($^t$butyldimethylsilyloxy)-1-butenylphosphonic acid diethyl ester (obtained in Step B of Example 3) (3.2 g, 10 mmol) in tetrahydrofuran (20 ml) at 20°C during 2 hours. Then, the reaction mixture is concentrated *in vacuo*, diluted with brine, extracted with ethyl acetate and the title product is obtained by flash chromatography on silica gel (18.5 g, 90%).

STEP B:

E-1-[4-(DIETHOXYPHOSPHORYL)BUT-3-ENYLOXY]-N-BENZOYL CYTOSINE

To a mixture of (4-hydroxy-1-butenyl)phosphonic acid diethyl ester (1.04 g, 5 mmol), 1-hydroxy-N-benzoylcytosine (1.1 g, 5 mmol) and triphenylphosphine (1.31 g, 5 mmol) in anhydrous tetrahydrofuran (3 ml) stirred at 0°C is added diethylazodicarboxylate (0.9 g, 5 mmol) and the resulting mixture is stirred overnight at 20°C. Then, the reaction mixture is concentrated *in vacuo* and the title product is purified by flash chromatography on silica gel (1.27 g, 62%).

STEP C:

E-1-[4-(DIETHOXYPHOSPHORYL)BUT-3-ENYLOXY]CYTOSINE

A mixture of E-1-[4-(diethoxyphosphoryl)but-3-enyloxy]-N-benzoyl cytosine (0.6 g, 1.5 mmol) in a saturated ethanol solution of hydrochloric acid (10 ml) hermetically closed is stirred overnight at 20°C. Then, the reaction mixture is concentrated *in vacuo* and the title product obtained by flash chromatography on silica gel (0.41 g, 90%).

STEP D:

E-1-[4-(DIHYDROXYPHOSPHORYL)BUT-3-ENYLOXY]CYTOSINE

A mixture of E-1-[4-(diethoxyphosphoryl)but-3-enyloxy]cytosine (0.305 g, 1 mmol) and trimethylsilylbromide (0.75 ml, 6 mmol) in anhydrous acetonitrile (5 ml) is stirred overnight at 20°C under argon. Then, the reaction mixture is heated with methanol (5 ml), concentrated *in vacuo*, diluted with absolute ethanol to afford the title product on cooling (0.18 g, 70%).

**EXAMPLE 9**

**E-1-[4-(DIHYDROXYPHOSPHORYL)BUT-3-ENYLOXY]THYMINE**

STEP A:

E-1-[4-(DIETHOXYPHOSPHORYL)BUT-3-ENYLOXY]THYMINE

To a mixture of 4-hydroxy-1-butenylphosphonic acid diethylester (1.04 g, 5 mmol), 1-hydroxythymine (0.72 g, 5 mmol), and triphenylphosphine (1.31 g, 5 mmol) in anhydrous tetrahydrofuran (30 ml) stirred at 0°C is added diethylazodicarboxylate (0.9 g, 5 mmol) and the mixture is stirred overnight at 20°C. Then, the reaction mixture is concentrated *in vacuo* and the title product is purified by flash chromatography on silica gel (1.08 g, 65%).

STEP B:

E-1-[4-(DIHYDROXYPHOSPHORYL)BUT-3-ENYLOXY]THYMINE

A mixture of E-1-[4-(diethoxyphosphoryl)but-3-enyloxy]thymine (1.0 g, 3 mmol) and trimethylsilyl bromide (2.2 ml, 18 mmol) in anhydrous acetonitrile (15 ml) is stirred overnight at 20°C under argon. Then, the reaction mixture is treated with methanol, concentrated in vacuo, and diluted with absolute ethanol to give the title product on cooling (0.47 g, 55%).

**EXAMPLE 10**

**E-9-[5-(DIHYDROXYPHOSPHORYL)-3-**HYDROXYMETHYL-4-PENTENYL]**GUANINE**

STEP A:

1-BROMO-2-$^t$BUTYLDIMETHYLSILYLOXY ETHANE

To a mixture of $^t$butyldimethylsilylchloride (42.4 g, 280 mmol) and 1,8-diazabicyclo-5,4-undec-7-ene (42.8 g, 280 mmol) in anhydrous dichloromethane under argon at 20°C, was added slowly 2-bromo-1-ethanol (32 g, 255 mmol). The reaction mixture was stirred 4 hours at 20°C, then concentrated *in vacuo*, diluted with brine and extracted with diethylether. The title product was purified by flash chromatography on silica gel (57 g, 95%).

STEP B:

4-$^t$BUTYLDIMETHYLSILYLOXY-2-ETHOXYCARBONYL BUTANOIC ACID ETHYL ESTER

A solution of 1-bromo-2-$^t$butyldimethylsilyloxy ethane (57 g, 238 mmol) in anhydrous tetrahydrofuran (200 ml) was added dropwise to a mixture obtained with sodium hydride (13.9 g, 60% in oil, 334 mmol) and diethylmalonate (53.5 g, 334 mmol) in anhydrous tetrahydrofuran (300 ml) at 0°C under argon. Then, the reaction mixture was stirred under reflux overnight under argon. Then, the reaction mixture was concentrated *in vacuo*, diluted with a saturated aqueous solution of ammonium chloride, extracted with diethyl ether and the title product was purified by flash chromatography (64 g, 85%).

STEP C:

4-$^t$BUTYLDIMETHYLSILYLOXY-2-HYDROXYMETHYL-1-BUTANOL

A solution of 4-$^t$butyldimethylsilyloxy-2-ethoxycarbonyl butanoic acid ethyl ester (32.0 g, 100 mmol) in anhydrous diethyl ether (150 ml) was added dropwise to a suspension of lithium aluminum hydride (7.8 g, 200 mmol) in anhydrous diethylether (200 ml) at 15°C under argon. The reaction mixture was stirred 2½ hours at

-15°C and then hydrolized by sequential addition of water (7.8 ml), 3.75 M NaOH (7.8 ml) and water (23.3 ml). The resulting suspension was filtered off, washed with diethylether and the title product was obtained by flash chromatography on silica gel (29.5 g, 62%).

STEP D:

4-$^t$BUTYLDIMETHYLSILYLOXY-2-ACETYLOXYMETHYL-1-BUTANOL

To a mixture of 4-$^t$butyldimethylsilyloxy-2-hydroxymethyl-1-butanol (9.86 g, 40 mmol), triethylamine (5.7 ml, 40 mmol) and dimethylamino pyridine (0.5 g, 4 mmol) in anhydrous dichloromethane (100 ml), acetic anhydride (3,9 ml, 40 mmol) was added. The reaction mixture was stirred overnight at 20°C, concentrated *in vacuo* and the title product obtained by flash chsromatography on silica gel (7.00 g, 60%).

STEP E:

4-$^t$BUTYLDIMETHYLSILYLOXY-2-ACETYLOXYMETHYL-1-TETRAHYDROPYRANYLOXYBUTANE

A mixture of 4-$^t$butyldimethylsilyloxy-2-acetyloxymethyl-1-butanol (5.5 g, 20 mmol),dihydropyran (1.68 g, 20 mmol) and p-toluene sulfonic acid (0.1 g) in anhydrous dichloromethane (50 ml) was stirred 2 hours at 20°C. Then, the reaction mixture was concentrated *in vacuo* and the title product purified by flash chromatography on silica gel (6.2 g, 86%).

STEP F:

4-$^t$BUTYLDIMETHYLSILYLOXY-2-HYDROXYMETHYL-1-TETRAHYDROPYRANYLOXYBUTANE

A mixture of 4-$^t$butyldimethylsilyloxy-2-acetyloxymethyl-1-tetrahydropyranyloxybutane (3.6 g, 10 mmol), sodium methylate (0.25 g, 5 mmol) in absolute methanol was stirred overnight at 20°C. Then, the reaction mixture was concentrated *in vacuo*, diluted with brine and extracted with dichloromethane. The title product was purified by flash chromatography on silica gel (25 g, 80%).

STEP G:

4-$^t$BUTYLDIMETHYLSILYLOXY-2-TETRAHYDROPYRANYLOXYMETHYL BUTANAL

A mixture of 4-$^t$butyldimethylsilyloxy-2-hydroxymethyl-1-tetrahydropyranyloxybutane (2.25 g, 7 mmol), molecular sieves in powder, N-methylmorpholine-N-oxyde (4.4 g, 10 mmol) and TPAP (0.12 g, 0.35 mmol) in anhydrous dichloromethane (70 ml) was stirred overnight at 20°C. Then, the reaction mixture was filtered trough celite concentrated in vacuo and the title product was purified by flash chromatography on silica gel (1.32 g, 60%).

STEP H:

(R,S)E-(5-$^t$BUTYLDIMETHYLSILYLOXY-3-TETRAHYDROPYRANYLOXYMETHYL-1-PENTENYL)PHOSPHONIC ACID DIETHYL ESTER

A solution of 1.6M n-butyllithium in hexane (3 ml, 4.6 mmol) was added to a solution of tetraethylmethylene bisphosphonate (1.32 g, 4.6 mmol) in anhydrous tetrahydrofuran (50 ml) at -78°C. After 30 minutes, a solution of 4-$^t$butyldimethylsilyloxy-2-tetrahydropyranyloxymethylbutanal (1.08 g, 3.3 mmol) in anhydrous tetrahydrofuran (10 ml) was added dropwise. The reaction mixture was stirred 3 hours at -78°C and overnight at 20°C, then hydrolized with brine and extracted with diethylether. The product was isolated by flash chromatography on silica gel (1.2 g, 82%).

STEP I:

(R,S)-E-(5-HYDROXY-3-TETRAHYDROPYRANYLOXYMETHYL-1-PENTENYL)PHOSPHONIC ACID DIETHYL ESTER

A mixture of (R,S)-E-(5-$^t$butyldimethylsilyloxy-3-tetrahydropyranyloxymethyl-1-pentenyl)phosphonic acid diethyl ester (1.2 g, 2.65 mmol) and 1M-tetrabutylammonium fluoride in tetrahydrofuran (4 ml, 4 mmol) was stirred 2 hours at 20°C. Then, the reaction mixture was concentrated *in vacuo* and the product obtained by flash chromatography on silica gel (0.7 g, 80%).

STEP J:

(R,S)-E-(5-TOSYLOXY-3-TETRAHYDROPYRANYLOXYMETHYL-1-PENTENYL)PHOSPHONIC ACID DIETHYL ESTER

A mixture of (R,S)-E-(5-hydroxy-3-tetrahydropyranyloxymethyl-1-pentenyl)phosphonic acid diethyl ester (0.65 g, 1.9 mmol), triethylamine (0.35 g, 2.5 mmol), tosylchloride (0.475 g, 2.5 mmol) and dimethylaminopyridine (0.005 g, 0.04 mmol) in anhydrous dichloromethane was stirred 4 hours at 20°C. Then, the reaction mixture was concentrated *in vacuo* and the title product obtained by flash chromatography on silica gel (0.9 g, 94%).

STEP K:

(R,S)-E-9-[5-(DIETHOXYPHOSPHORYL)-3-(TETRAHYDROPYRANYLOXYMETHYL)-PENT-4-ENYL]-6-CHLORO-2-AMINOPURINE

A mixture of 6-chloro-2-aminopurine (0.34 g, 2 mmol), sodium hydride (0.075 g, 2 mmol , 50% in oil) in anhydrous dimethylformamide (5 ml) was stirred 10 minutes at 20°C under argon. Then, a solution of (R,S)-E-5-tosyloxy-3-tetrahydropyranyloxymethyl-1- pentenylphosphonic acid diethyl ester (0.9 g, 1.8 mmol) in anhydrous dimethylformamide (3 ml) was added and the resulting mixture was stirred overnight at 20°C. Then, the reaction mixture was concentrated *in vacuo*, diluted with brine, extracted with dichloromethane and the title product purified by flash chromatography on silica gel (0.4 g, 46%).

STEP L:

(R,S)-E-9-[5-(DIHYDROXYPHOSPHORYL)-3-(TETRAHYDROPYRANYLOXYMETHYL)-PENT-4-ENYL]-6-CHLORO-2-AMINOPURINE

A mixture of (R,S)-E-9-[5-diethoxyphosphoryl)-3-(tetrahydropyranyloxy methyl)-pent-4-enyl]-6-chloro-2-aminopurine (0.39 g, 0.8 mmol) and trimethylsilylbromide (0.52 ml, 4 mmol) in anhydrous acetonitrile (2 ml) was stirred overnight at 20C. Then, the reaction mixture was treated with methanol (5 ml) and concentrated *in vacuo*. The crude product (~0.35 g) was used for the next step without further purification.

STEP M:

E-9-[5-(DIHYDROXYPHOSPHORYL)-3-HYDROXYMETHYL-4-PENTENYL]GUANINE

A mixture of crude 4-$^t$butyldimethylsilyloxy-2-hydroxymethyl-1-butanol (0.35 , ~0.8 mmol) in 1N hydrochloric acid (5 ml) was heated under reflux overnight. Then, the reaction mixture was concentrated *in vacuo* and diluted with absolute ethanol. The title product crystallized on cooling (0.16 g, 62%).

**EXAMPLE 11**

**<u>(R,S)-E-9-[5-(DIHYDROXYPHOSPHORYL)-3-(HYDROXYMETHYL)-4-PENTENYL]ADENINE</u>**

STEP A:

(R,S)-E-9-[5-(DIETHOXYPHOSPHORYL)-3-(TETRAHYDROPYRANYLOXYMETHYL)-4-PENTENYL]ADENINE

By using the procedure described in Step K, Example 10 with adenine (0.27 g) the title product is obtained (0.39 g, 55%).

STEP B:

(R,S)-E-9-[5-(DIETHOXYPHOSPHORYL)-3-HYDROXYMETHYL-4-PENTENYL]ADENINE

A mixture of (R,S)-E-9-[5-(diethoxyphosphoryl)-3-(tetrahydropyranyloxymethyl)-4-pentenyl]adenine (0.39 g, 0.86 mmol) and p-toluene sulfonic acid ((0.05 g) in ethanol is stirred overnight at 20°C. Then, the reaction mixture is concentrated *in vacuo*, diluted with dichloromethane and washed with brine. The title product is obtained by flash chromatography on silica gel (0.285 g, 90%).

STEP C:

(R,S)-E-9-[5-(DIHYDROXYPHOSPHORYL)-3-(HYDROXYMETHYL)-4-PENTENYL]ADENINE

A mixture of (R,S)-E-9-[5-(diethoxyphosphoryl)-3-(hydroxymethyl)-4-pentenyl]adenine (0.285 g, 0.77 mmol) and trimethylsilylbromide (0.75 g, 6 mmol) in anhydrous acetonitrile (5 ml) is stirred overnight at 20°C under argon. Then, the reaction mixture is treated with methanol, concentrated *in vacuo* and diluted with absolute ethanol. The title product is crystallized on cooling (0.18 g, 75%).

**EXAMPLE 12**

**(R,S)-E-1-(5-DIHYDROXYPHOSPHORYL-3-HYDROXYMETHYL-4-PENTENYL)CYTOSINE**

STEP A:

(R,S)-E-(5-IODO-3-TETRAHYDROPYRANYLOXY-1-PENTENYL)PHOSPHONIC ACID DIETHYL ESTER

By using the procedure described in Step D, Example 1 with E-5-tosyloxy-3-tetrahydropyranyloxymethyl-1-butenylphosphonic acid diethyl ester (2.85 g, 5 mmol), described in Step J, Example 10, the title product is obtained (1.95 g, 85%).

STEP B:

(R,S)-E-1-(5-DIETHOXYPHOSPHORYL-3-TETRAHYDROPYRANYLOXYMETHYL-4-PENTENYL)-N-ACETYLCYTOSINE

By using the procedure described in Step E, Example 1 with (R,S)-E-(5-iodo-3-tetrahydropyranyloxy-1-pentenyl)phosphonic acid diethyl ester (0.91 g, 2 mmol) the title product is obtained (0.72 g, 75%).

STEP C:

(R,S)-E-1-(5-DIETHOXYPHOSPHORYL-3-TETRAHYDROPYRANYLOXYMETHYL-4-PENTENYL)CYTOSINE

By using the procedure described in Step F, Example 1 with (R,S)-E-1-(5-diethoxyphosphoryl-3-tetrahydropyranyloxymethyl-4-pentenyl)-N-acetylcytosine (0.7 g, 1.45 mmol) the title product is obtained (0.575 g, 90%).

STEP D:

(R,S)-E-1-(5-DIETHOXYPHOSPHORYL-3-HYDROXYMETHYL-4-PENTENYL)CYTOSINE

By using the procedure described in Step B, Example 11 with (R,S)-E-1-(5-diethoxyphosphoryl-3-tetrahydropyranyloxymethyl-4-pentenyl)cytosine (0.53 g, 1.2 mmol) the title product is obtained (0.37 g, 85%).

STEP E:

(R,S)-E-1-(5-DIHYDROXYPHOSPHORYL-3-HYDROXYMETHYL-4-PENTENYL)CYTOSINE

By using the procedure described in Step C, Example 11 with (R,S)-E-1-(5-diethoxyphosphoryl-3-hydroxymethyl-4-pentenyl)cytosine (0.36 g, 1 mmol) the title product is obtained (0.22 g, 75%).

**EXAMPLE 13**

**(R,S)-E-1-(5-DIHYDROXYPHOSPHORYL-3-HYDROXYMETHYL-4-PENTENYL)THYMINE**

STEP A:

(R,S)-E-1-(5-DIETHOXYPHOSPHORYL-3-DIHYDROPYRANYLOXYMETHYL-4-PENTENYL)THYMINE

By using the procedure described in Step A, Example 2 with (R,S)-E-(5-iodo-3-tetrahydropyranyloxy-1-pentenyl)phosphonic acid diethyl ester (0.91 g, 2 mmol), described in Step A, Example 12, the title product is obtained (0.41 g, 45%).

STEP B:

(R,S)-E-1-(5-DIETHOXYPHOSPHORYL-3-HYDROXYMETHYL-4-PENTENYL)THYMINE

By using the procedure described in Step B, Example 11 with (R,S)-E-1-(5-diethoxyphosphoryl-3-dihydropyranyloxymethyl-4-pentenyl)thymine (0.4 g, 0.87 mmol) the title product is obtained (0.29 g, 90%).

STEP C:

<u>(R,S)-E-1-(5-DIHYDROXYPHOSPHORYL-3-HYDROXYMETHYL-4-PENTENYL)THYMINE</u>

By using the procedure described in Step B, Example 2 with (R,S)-E-1-(5-diethoxyphosphoryl-3-hydroxymethyl-4-pentenyl)thymine (0.25 g, 0.67 mmol) the title product is obtained (0.175 g, 82%)

**EXAMPLE 14**

**<u>(R,S)-E-9-[(3-DIHYDROXYPHOSPHORYL-1-HYDROXYMETHYL-2-PROPENYLOXY)METHYL]GUANINE</u>**

STEP A:

1-t.BUTYLDIPHENYLSILYLOXY-2-PROPENE

To a mixture of allylalcohol (29 g, 500 mmol) and triethylamine (50.5 g, 500 mmol) in anhydrous dichloromethane (500 ml) is added dropwise a solution of t-butyldiphenylsilyl chloride (137 g, 500 mmol) in anhydrous dichloromethane (150 ml) at 20°C. The reaction mixture is stirred 20 hours at 20°C, then washed with brine and the product is obtained by flash chromatography on silica gel (111 g, 84%).

STEP B:

2-t.BUTYLDIPHENYLSILYLOXYACETALDEHYDE

Ozone is bubbled into a mixture of 1-t.butyldiphenylsilyloxy-2-propene (60 g, 200 mmol) in anhydrous dichloromethane (200 ml) at -78°C. After apparition of the blue coloration, a solution of dimethylsulfide (12.4 g, 200 mmol) in dichloromethane (30 ml) is added. Then, the reaction mixture is washed with brine and the title product is obtained by flash chromatography on silica gel (38.8 g, 65%).

STEP C:

(R,S)-1-t.BUTYLDIPHENYLSILYLOXY-2-HYDROXY-3-BUTENE

A 1M solution of vinyl magnesium bromide in tetrahydrofuran (110 ml, 110 mmol) is added dropwise to a mixture of 2-t.butyldiphenylsilyloxyacetaldehyde (30 g, 100 mmol) in anhydrous tetrahydrofuran (200 ml) at 0°C. Then, the reaction mixture is stirred overnight at 29°C, hydrolized with brine, concentrated *in vacuo* and extracted with diethylether. The title product is obtained by flash chromatography on silica gel (24.4 g, 75%).

STEP D:

(R,S)-1-t.BUTYLDIPHENYLSILYLOXY-2-TETRAHYDROPYRANYLOXY-3-BUTENE

A mixture of (R,S)-1-t.butyldiphenylsilyloxy-2-hydroxy-3-butene (24.4 g, 75 mmol), dihydropyran (4.63 g, 75 mmol) and p-toluene sulfonic acid (1 g) in anhydrous dichloromethane (200 ml) is stirred overnight at 20°C. Then, the reaction mixture is washed with brine and the title product is obtained by flash chromatography on silica gel (26 g, 85%).

STEP E:

(R,S)-3-t.BUTYLDIPHENYLSILYLOXY-2-TETRAHYDROPYRANYLOXYPROPIONALDEHYDE

Ozone is bubbled into a mixture of 2-t.butyldiphenylsilyloxyacetaldehyde using (R,S)-1-t.butyldiphenylsilyloxy-2-tetrahydropyranyloxy-3-butene (26 g, 63 mmol) in anhydrous dichloromethane at -78°C until saturation of the solution. Then, nitrogen is bubbled into the mixture and the ozonide is reduced by an excess of dimethylsulfide. Then, the reaction mixture is washed with brine, dried and concentrated in vacuo. The crude product (18 g, ~70%) is used for the next step without further purification.

STEP F:

(R,S)-E-(4-t.BUTYLDIPHENYLSILYLOXY-3-TETRAHYDROPYRANYLOXY-1-BUTENYL)PHOSPHONIC ACID DIETHYL ESTER

By using the procedure described in Step H of Example 10 with (R,S)-3-t.butyldiphenylsilyloxy-2-tetrahydropyranyloxypropionaldehyde (18 g, 44 mmol) the title product is obtained (19.2 g, 80%).

STEP G:

(R,S)-E-(4-t.BUTYLDIPHENYLSILYLOXY-3-HYDROXY-1-BUTENYL)PHOSPHONIC ACID DIETHYL ESTER

By using the procedure described in Step C of Example 5 with (R,S)-E-(4-t.butyldiphenylsilyloxy-3-tetrahydropyranyloxy-1-butenyl)phosphonic acid diethyl ester (19 g, 35 mmol) the title product is obtained (14.5 g, 90%).

STEP H:

(R,S)-E-(4-t.BUTYLDIPHENYLSILYLOXY-3-CHLOROMETHOXY-1-BUTENYL)PHOSPHONIC ACID DIETHYL ESTER

By using the procedure described in Step D of Example 5 with (R,S)-E-(4-t.butyldiphenylsilyloxy-3-hydroxy-1-butenyl)phosphonic acid diethyl ester (14.5 g, 31 mmol) the title product is obtained and used in a 1M solution of 1,2-dichloroethane in the next step without further purification.

STEP I:

(R,S)-E-9-[(3-DIETHOXYPHOSPHORYL-1-t.BUTYLPHENYLSILYLOXYMETHYL-2-PROPENYLOXY)METHYL] GUANINE

By using the procedure described in Step E of Example 5 with guanine (3.80 g, 25 mmol) and (R,S)-E-(4-t.butyldiphenylsilyloxy-3-chloromethoxy-1-butenyl)phosphonic acid diethyl ester (25 ml of a 1M solution in 1,2-dichloroethane, 25 mmol), the title product is obtained (4.7 g, 30%).

STEP J:

(R,S)-E-9-[(3-DIETHOXYPHOSPHORYL-1-HYDROXYMETHYL-2-PROPENYLOXY)METHYL]GUANINE

A mixture of (R,S)-E-9-[(3-diethoxyphosphoryl-1-t.butylphenylsilyloxy-methyl-2-propenyloxy)methyl]guanine (3.75 g, 6 mmol) and a 1M solution of tetrabutylammonium fluoride (7 ml, 7 mmol) in tetrahydrofuran is stirred overnight at 20°C. Then, the reaction mixture is concentrated *in vacuo*, diluted with dichloromethane, washed with brine and the title product is obtained by recrystallization in ethyl acetate/ethanol (1.85 g, 80%).

STEP K:

(R,S)-E-9-[(3-DIHYDROXYPHOSPHORYL-1-HYDROXYMETHYL-2-PROPENYLOXY)METHYL]GUANINE

A mixture of (R,S)-E-9-[(3-diethoxyphosphoryl-1-hydroxymethyl-2-propenyloxy)methyl]guanine (1.73 g, 4.5 mmol) and trimethylsilylbromide (4.5 g, 25 mmol) in anhydrous acetonitrile is stirred overnight at 20°C under argon. Then, the reaction mixture is treated with methanol, concentrated *in vacuo*, and diluted with absolute ethanol. The title product is crystallized on cooling (1.04 g, 70%).

**EXAMPLE 15**

**(R,S)-E-9-[(3-DIHYDROXYPHOSPHORYL-1-HYDROXYMETHYL-2-PROPENYLOXY)METHYL]ADENINE**

STEP A:

(R,S)-E-9-[(3-DIETHOXYPHOSPHORYL-1-t.BUTYLDIPHENYLSILYLOXYMETHYL-2-PROPENYLOXY)METHYL] ADENINE

By using the procedure described in Step A of Example 7 with (R,S)-E-4-t.butyldiphenylsilyloxy-3-chloromethoxy-1-butenyl phosphonic acid diethyl ester (25 ml of a 1M solution, 25 mmol), the title product is obtained (6.85 g, 45%).

STEP B:

(R,S)-E-9-[(3-DIETHOXYPHOSPHORYL-1-HYDROXYMETHYL-2-PROPENYLOXY)METHYL]ADENINE

A mixture of (R,S)-E-9-[(3-diethoxyphosphoryl-1-t.butyldiphenylsilyloxymethyl-2-propenyloxy)methyl] adenine (6.1 g, 10 mmol) and a 1M solution of tetrabutylammonium fluoride (11 ml, 11 mmol) in tetrahydrofuran is stirred overnight at 20°C. Then, the reaction mixture is concentrated *in vacuo*, diluted with dichloromethane, washed with brine and the title product is obtained by flash chromatography on silica gel (2.9 g, 75%).

STEP C:

(R,S)-E-9-[(3-DIHYDROXYPHOSPHORYL-1-HYDROXYMETHYL-2-PROPENYLOXY)METHYL]ADENINE

By using the procedure described in Step B of Example 7 with (R,S)-E-9-[(3-diethoxyphosphoryl-1-hydroxymethyl-2-propenyloxy)methyl]adenine (2.3 g, 6 mmol), the title product is obtained (1.1 g, 55%).

**EXAMPLE 16**

**(R,S)-E-1-[(3-DIHYDROXYPHOSPHORYL-1-HYDROXYMETHYL-2-PROPENYLOXY)METHYL]CYTOSINE**

STEP A:

(R,S)-E-1-[(3-DIETHOXYPHOSPHORYL-1-t.BUTYLDIPHENYLSILYLOXYMETHYL-2-PROPENYLOXY)METHYL] N-ACETYLCYTOSINE

By using the procedure described in Step E of Example 5 with (R,S)-E-(4-t.butyldiphenylsilyloxy-3-chloromethoxy-1-butenyl)phosphonic acid diethyl ester (25 ml, 1M solution in 1,2-dichloroethane , 25 mmol), the title product is obtained (7.15 g, 45%).

STEP B:

(R,S)-E-1-[(3-DIETHOXYPHOSPHORYL-1-t.BUTYLDIPHENYLSILYLOXYMETHYL-2-PROPENYLOXY)METHYL] CYTOSINE

By using the procedure described in Step F of Example 5 with (R,S)-E-1-[(3-diethoxyphosphoryl-1-t.butyldiphenylsilyloxymethyl-2-propenyloxy)methyl]N-acetylcytosine (6.4 g, 10 mmol), the title product is obtained (4.4 g, 75%).

STEP C:

(R,S)-E-1-[(3-DIETHOXYPHOSPHORYL-1-HYDROXYMETHYL-2-PROPENYLOXY)METHYL]CYTOSINE

By using the procedure described in Step B of Example 15 with (R,S)-E-1-[(3-diethoxyphosphoryl-1-t.butyldiphenylsilyloxymethyl-2-propenyloxy)methyl]cytosine (2.92 g, 5 mmol), the title product is obtained (1.2 g, 70%).

STEP D:

(R,S)-E-1-[(3-DIHYDROXYPHOSPHORYL-1-HYDROXYMETHYL-2-PROPENYLOXY)METHYL]CYTOSINE

By using the procedure described in Step G of Example 5 with (R,S)-E-1-[(3-diethoxyphosphoryl-1-hydroxymethyl-2-propenyloxy)methyl]cytosine (1.64 g, 3 mmol), the title product is obtained (0.64 g, 70%).

**EXAMPLE 17**

**(R,S)-E-1-[(3-DIHYDROXYPHOSPHORYL-1-HYDROXYMETHYL-2-PROPENYLOXY)METHYL]THYMINE**

STEP A:

(R,S)-E-1-[(3-DIETHOXYPHOSPHORYL-1-t.BUTYLDIPHENYLSILYLOXYMETHYL-2-PROPENYLOXY)METHYL] THYMINE

By using the procedure described in Step A of Example 6 with (R,S)-E-(4-t.butyldiphenylsilyloxy-3-chloromethoxy-1-butenyl)phosphonic acid diethyl ester (25 ml of a 1M solution in 1,2- dichloroethane, 25 mmol), the title product is obtained (6.75 g, 45%).

STEP B:

(R,S)-E-1-[(3-DIETHOXYPHOSPHORYL-1-HYDROXYMETHYL-2-PROPENYLOXY)METHYL]THYMINE

By using the procedure described in Step B of Example 15,with (R,S)-E-1-[(3-diethoxyphosphoryl-1-t.butyldiphenylsilyloxymethyl-2-propenyloxy)methyl]thymine(6 g, 10 mmol), the title product is obtained (2.5 g, 70%).

STEP C:

(R,S)-E-1-[(3-DIHYDROXYPHOSPHORYL-1-HYDROXYMETHYL-2-PROPENYLOXY)METHYL]THYMINE

By using the procedure described in Step B of Example 6 with (R,S)-E-1-[(3-diethoxyphosphoryl-1-hydroxymethyl-2-propenyloxy)methyl]thymine (2.2 g, 6 mmol), the title product is obtained (1.1 g, 60%).

**EXAMPLE 18**

**(R,S)-E-1-[4-(DIHYDROXYPHOSPHORYL)-2-(HYDROXYMETHYL)-3-BUTENYL]CYTOSINE**

STEP A:

2-$^t$BUTYLDIMETHYLSILYLOXYMETHYL-2-PROPEN-1-OL

A solution of $^t$butyldimethylsilyl chloride (30.8 g, 200 mmol) in anhydrous dichloromethane (100 ml) is added dropwise to a cooled (0°C) mixture of 2-hydroxymethyl-2-propen-1-ol (17.6 g, 200 mmol), triethylamine (30 ml) and 4-dimethylaminopyridine (2.5 g) in anhydrous dichloromethane (400 ml). The reaction mixture is stirred 20 hours at 20°C, washed with aqueous saturated ammonium chloride and the title product is obtained by flash chromatography on silica gel (31 g, 75%).

STEP B:

2-$^t$BUTYLDIMETHYLSILYLOXYMETHYL-1-TETRAHYDROPYRANYLOXY-2-PROPENE

A mixture of 2-$^t$butyldimethylsilyloxymethyl-2-propen-1-ol (31 g, 150 mmol), dihydropyran (13.2 g, 150 mmol) and p-toluene sulfonic acid (0.5 g) in anhydrous dichloromethane (200 ml) is stirred overnight at 20°C. Then, the reaction mixture is concentrated *in vacuo* and the product is obtained by flash chromatography on silica gel (32 g, 85%).

STEP C:

(R,S)-2-$^t$BUTYLDIMETHYLSILYLOXYMETHYL-1-TETRAHYDROPYRANYLOXY-1-PROPANOL

To a mixture of 2-$^t$butyldimethylsilyloxymethyl-1-tetrahydropyranyloxy-2-propene (30 g, 120 mmol) in anhydrous tetrahydrofuran (300 ml), sodium borohydride (2.7 g, 70 mmol) is added at once. Then, a solution of acetic acid (4.2 g, 70 mmol) in anhydrous tetrahydrofuran (15 ml) is added carefully and throughout the reaction sequence the temperature is kept in the 10-20°C range. Then, the reaction mixture is stirred 2 hours at 20°C, hydrolized with 2M sodium hydroxide, concentrated *in vacuo* and extracted with dichloromethane. The title product is obtained by flash chromatography on silica gel (24 g, 75%).

STEP D:

(R,S)-2-$^t$BUTYLDIMETHYLSILYLOXYMETHYL-3-TETRAHYDROPYRANYLOXYPROPANAL

A mixture of (R,S)-2-$^t$butyldimethylsilyloxymethyl-1-tetrahydropyranyloxy-1-propanol (15.5 g, 50 mmol),molecular sieves in powder, N-methylmorpholine-N-oxyde (28.5 g, 65 mmol) and TPAP (0.35 g, 1 mmol) in anhydrous dichloromethane (300 ml) is stirred overnight at 20°C. Then, the reaction mixture is filtered on celite, concentrated *in vacuo* and the title product is purified by flash chromatography on silica gel (10 g, 65%).

STEP E:

(R,S)-E-[4-($^t$BUTYLDIMETHYLSILYLOXYMETHYL)-3 -(TETRAHYDROPYRANYLOXY)-1-BUTENYL]PHOSPHONIC ACID DIETHYL ESTER

A solution of 1.6M of n-butyllithium (3 ml, 4.5 mmol in hexane) is added to a mixture of tetraethylmethylenebisphosphonate (1.3 g, 4.6 mmol) in anhydrous tetrahydrofuran (50 ml) at -78°C under argon. After 30 minutes, a solution of (R,S)-2-$^t$butyldimethylsilyloxy-3-tetrahydropyranyloxypropanal (1.05 g, 3.3 mmol) in anhydrous tetrahydrofuran (10 ml) is added dropwise and the resulting reaction mixture is stirred 3 hours at -78°C and allowed to warm at 20°C overnight. Then, the reaction is quenched with aqueous ammonium chloride and concentrated *in vacuo* and extracted with diethyl ether. The title product is obtained by flash chromatography on silica gel (1.4 g, 85%).

STEP F:

(R,S)-E-(4 -HYDROXYMETHYL-3-TETRAHYDROPYRANYLOXY-1-BUTENYL)PHOSPHONIC ACID DIETHYL ESTER

A mixture of (R,S)-E-[4-([t]butyldimethylsilyloxymethyl)-3-(tetrahydropyranyloxy)-1-butenyl]phosphonic acid diethyl ester (1.1 g,2 mmol) and 1M tetrabutylammonium fluoride (3.5 ml, 3.5 mmol) in tetrahydrofuran is stirred 3 hours at 20°C. Then, the reaction mixture is concentrated *in vacuo* and the title product obtained by flash chromatography on silica gel (0.69 g, 90%).

STEP G:

(R,S)-E-(4-MESYLOXYMETHYL-3-TETRAHYDROPYRANYLOXY-1-BUTENYL)PHOSPHONIC ACID DIETHYL ESTER

To a mixture of (R,S)-E-(4-hydroxymethyl-3-tetrahydropyranyloxy-1-butenyl)phosphonic acid diethyl ester (0.69 g, 1.8 mmol) triethylamine (0.3 ml, 2 mmol) and dimethylamino pyridine (0.1 g) in anhydrous dichloromethane (10 ml),mesyl chloride is added (0.23 g, 2 mmol) at -10°C. Then, the reaction mixture is stirred overnight at 20°C, concentrated *in vacuo* and the title product is obtained by flash chromatography on silica gel (0.67 g, 80%).

STEP H:

(R,S)-E-(4-IODO-3-TETRAHYDROPYRANYLOXYMETHYL-1-BUTENYL)PHOSPHONIC ACID DIETHYL ESTER

A mixture of (R,S)-E-(4 -mesyloxymethyl- 3-tetrahydropyranyloxy-1-butenyl)phosphonic acid diethyl ester (0.67 g, 1.44 mmol) and sodium iodide (0.24 g, 1.6 mmol) in acetone (10 ml) is heated under reflux overnight. Then, the reaction mixture is concentrated *in vacuo*, diluted with diethyl ether, washed with brine and the title product is obtained by flash chromatography on silica gel (0.64 g, 90%).

STEP I:

(R,S)-E-1-[4-(DIETHOXYPHOSPHORYL)-2-TETRAHYDROPYRANYLOXYMETHYL-3-BUTENYL]-N-ACETYLCYTOSINE

A mixture of N-acetylcytosine (0.22 g, 1.5 mmol), (R,S)-E-(4-iodo-3-tetrahydropyranyloxymethyl-1-butenyl)phosphonic acid diethyl ester (0.64 g, 1.3 mmol) and potassium carbonate (0.21 g, 1.5 mmol) in anhydrous dimethylformamide (5 ml) is stirred at 10°C for 2 days. Then, the reaction mixture is concentrated *in vacuo*, diluted with brine and extracted with dichloromethane. The title product is obtained by flash chromatography on silica gel (0.55 g, 70%).

STEP J:

(R,S)-E-1-[4-(DIETHOXYPHOSPHORYL)-2-TETRAHYDROPYRANYLOXYMETHYL-3-BUTENYL]CYTOSINE

A mixture of (R,S)-E-1-[4-(diethoxyphosphoryl)-2-tetrahydropyranyloxymethyl-3-butenyl]-N-acetylcytosine (0.52 g, 1. mmol) in a saturated ethanolic solution of ammonia (10 ml) hermetically closed, is stirred overnight at 20°C. Then, the reaction mixture is concentrated *in vacuo* and the title product is obtained by flash chromatography on silica gel (0.41 g, 85%).

STEP K:

(R,S)-E-1-[4-(DIETHOXYPHOSPHORYL)-2-HYDROXYMETHYL-3-BUTENYL]CYTOSINE

A mixture of (R,S)-E-1-[4-(diethoxyphosphoryl)-2-tetrahydropyranyloxymethyl-3-butenyl] cytosine (0.4 g, 0.84 mmol) and p-toluene sulfonic acid (0.05 g) in ethanol (10 ml) is stirred overnight at 20°C. Then, the reaction mixture is concentrated *in vacuo* diluted with dichloromethane, washed with brine and the title product is obtained by flash chromatography on silica gel (0.265 g, 80%).

STEP L:

(R,S)-E-1-[4-(DIHYDROXYPHOSPHORYL)-2-(HYDROXYMETHYL)-3-BUTENYL]CYTOSINE

A mixture of (R,S)-E-1-[4-(diethoxyphosphoryl)-2-hydroxymethyl-3-butenyl]cytosine (0.25 g, 0.63 mmol)

and trimethylsilybromide (0.5 ml, 4 mmol) in anhydrous acetonitrile (5 ml) is stirred overnight at 20°C under argon. Then, the reaction mixture is treated with methanol, concentrated *in vacuo*, diluted with absolute ethanol to give the crystallized title product on cooling (0.14 g, 65%).

**EXAMPLE 19**

**E-1-[4-(DIHYDROXYPHOSPHORYL)-2-HYDROXYMETHYL-3-BUTENYL]THYMINE**

STEP A:

(R,S)-E-1-[4-(DIETHOXYPHOSPHORYL)-2-(TETRAHYDROPYRANYLOXYMETHYL)-3-BUTENYL]THYMINE

A mixture of thymine (0.7 g, 5.5 mmol), (R,S)-E-(4-iodo-3-tetrahydropyranyloxymethyl-1-butenyl)phosphonic acid diethyl ester (2.5 g, 5 mmol), potassium carbonate (0.77 g, 5.5 mmol) in anhydrous dimethylformamide (50 ml) is stirred at 10°C for 3 days under argon. Then, the reaction mixture is concentrated *in vacuo*, diluted with brine and extracted with dichloromethane. The title product is obtained by flash chromatography on silica gel (0.86 g, 35%).

STEP B:

(R,S)-E-1-[4-(DIETHOXYPHOSPHORYL)-2-(HYDROXYMETHYL)-3-BUTENYL]THYMINE

A mixture of (R,S)-E-1-[4-(diethoxyphosphoryl)-2-(tetrahydropyranyloxymethyl)-3-butenyl]thymine (0.495 g, 1 mmol) and p-toluene sulfonic acid (0.1 g) in absolute ethanol is stirred at 20°C overnight. Then, the reaction mixture is concentrated *in vacuo*, diluted with dichloromethane and washed with brine. The title product is obtained by flash chromatography on silica gel (0.31 g, 75%).

STEP C:

E-1-[4-(DIHYDROXYPHOSPHORYL)-2-HYDROXYMETHYL-3-BUTENYL]THYMINE

A mixture of (R,S)-E-1-[4-(diethoxyphosphoryl)-2-(hydroxymethyl)-3-butenyl]thymine (0.285 g, 0.7 mmol) and trimethylsilylbromide (0.5 ml, 4 mmol) in anhydrous acetonitrile is stirred overnight at 20°C under argon. Then, the reaction mixture is treated with methanol, concentrated *in vacuo* and diluted with absolute ethanol. The title product crystallized on cooling (0.135 g, 55%).

**EXAMPLE 20**

**(R,S)-E-1-[4-(DIHYDROXYPHOSPHORYL)-2-HYDROXYMETHYL-BUT-3-ENYLOXY]CYTOSINE**

STEP A:

(R,S)-E-1-[4-(DIETHOXYPHOSPHORYL)-2-(TETRAHYDROPYRENYLOXYMETHYL)-BUT-3-ENYLOXY]-N-BENZOYLCYTOSINE

To a mixture of (R,S)-E-(4-hydroxymethyl-3-tetrahydropyranyloxy-1-butenyl)phosphonic acid diethyl ester (0.77 g, 2 mmol), 1-hydroxy-N-benzoylcytosine (0.46 g, 2 mmol) and triphenylphosphine (0.525 g, 2 mmol) in anhydrous tetrahydrofuran (20 ml) stirred at 0°C, is added diethylazodicarboxylate (0.35 g, 2 mmol) and the resulting mixture is stirred overnight at 20°C under argon. Then, the reaction mixture is concentrated *in vacuo* and the title product is obtained by flash chromatography on silica gel (0.78 g, 65%).

STEP B:

(R,S)-E-1-[4-(DIETHOXYPHOSPHORYL)-2-(HYDROXYMETHYL)-BUT-3-ENYLOXY]CYTOSINE

A mixture of (R,S)-E-1-[4-(diethoxyphosphoryl)-2-(tetrahydropyranyloxymethyl)-but-3-enyloxy]-N-benzoylcytosine (0.72 g, 1.2 mmol) in saturated ethanolic hydrochloric acid is stirred overnight at 20°C. Then, the reaction mixture is concentrated *in vacuo*, diluted with brine and extracted with dichloromethane. The title product is obtained by flash chromatography on silica gel (0.41 g, 85%).

STEP C:

(R,S)-E-1-[4-(DIHYDROXYPHOSPHORYL)-2-HYDROXYMETHYL-BUT-3-ENYLOXY]CYTOSINE

A mixture of (R,S)-E-1-[4-(diethoxyphosphoryl)-2-(hydroxymethyl)-but-3-enyloxy]cytosine (0.4 g, 1 mmol) and trimethylsilylbromide (0.75 ml, 6 mmol) in anhydrous acetonitrile (5 ml) is stirred overnight at 20°C under argon. Then, the reaction mixture is treated with methanol (5 ml), concentrated *in vacuo* and diluted in absolute ethanol. The title product crystallized on cooling (0.19 g, 55%).

**EXAMPLE 21**

**(R,S)-E-1-[4-(DIHYDROXYPHOSPHORYL)-2-HYDROXYMETHYL-BUT-3-ENYLOXY]THYMINE**

STEP A:

E-1-[4-(DIETHOXYPHOSPHORYL)-2-(TETRAHYDROPYRANYLOXYMETHYL)-BUT-3-ENYLOXY]THYMINE

To a mixture of (R,S)-E-(4-hydroxymethyl-3-tetrahydropyranyloxy-1-butenyl)phosphonic acid diethyl ester (0.39 g, 1 mmol), 1-hydroxythymine (0.14 g, 1 mmol) and triphenylphosphine (0.26 g, 1 mmol) in anhydrous tetrahydrofuran (10 ml) stirred at 0°C, diethylazodicarboxylate (0.175 g, 1 mmol) is added. The reaction mixture is stirred overnight at 20°C under argon and concentrated *in vacuo*. The title product is obtained by flash chromatography on silica gel (0.36 g, 70%).

STEP B:

(R,S)-E-1-[4-(DIETHOXYPHOSPHORYL)-2-HYDROXYMETHYL-BUT-3-ENYLOXY]THYMINE

A mixture of E-1-[4-(diethoxyphosphoryl)-2-(tetrahydropyranyloxymethyl)-but-3-enyloxy]thymine (0.33 g, 0.55 mmol) and p-toluene sulfonic acid (0.05 g) in ethanol (5 ml) is stirred overnight at 20°C. Then, the reaction mixture is concentrated *in vacuo* and the title product is obtained by flash chromatography on silica gel (0.18 g, 82%).

STEP C:

(R,S)-E-1-[4-(DIHYDROXYPHOSPHORYL)-2-HYDROXYMETHYL-BUT-3-ENYLOXY]THYMINE

A mixture of (R,S)-E-1-[4-(diethoxyphosphoryl)-2-hydroxymethyl-but-3-enyloxy]thymine (0.18 g, 0.45 mmol) and trimethylsilylbromide (0.37 ml, 3 mmol) in anhydrous acetonitrile is stirred overnight at 20°C under argon. Then, the reaction mixture is treated with methanol, concentrated *in vacuo* and diluted with absolute ethanol. The title product is crystallized on cooling (0.085 g, 55%).

**EXAMPLE 22**

**(R,S)-E-9-(4-DIHYDROXYPHOSPHORYL-2-HYDROXY-3-BUTENYL)GUANINE**

STEP A:

(R,S)-E-(4-HYDROXY-3-TETRAHYDROPYRANYLOXY-1-BUTENYL)PHOSPHONIC ACID DIETHYL ESTER

By using the procedure described in Step A, Example 8, with (R,S)-E-4-t.butyldiphenylsilyloxy-3-tetrahydropyranyloxy-1-butenyl phosphonic acid diethyl ester (11 g, 20 mmol), (described in Step F, Example 14), the title product is obtained (5.2 g, 85%).

STEP B:

(R,S)-E-(4-MESYLOXY-3-TETRAHYDROPYRANYLOXY-1-BUTENYL)PHOSPHONIC ACID DIETHYL ESTER

By using the procedure described in Step G of Example 18 with (R,S)-E-(4-hydroxy-3-tetrahydropyranyloxy-1-butenyl)phosphonic acid diethyl ester (4.6 g, 15 mmol), the title product is obtained (5.05 g, 87%).

STEP C:

(R,S)-E-(4-IODO-3-TETRAHYDROPYRANYLOXY-1-BUTENYL)PHOSPHONIC ACID DIETHYL ESTER

By using the procedure described in Step H of Example 18 with (R,S)-E-(4-mesyloxy-3-tetrahydropyranyloxy-1-butenyl)phosphonic acid diethyl ester (4.6 g, 12 mmol), the title product is obtained (4.25 g, 85%).

STEP D:

(R,S)-E-9-(4-DIETHOXYPHOSPHORYL-2-TETRAHYDROPYRANYLOXY-1-BUTENYL)-6-CHLORO-2-AMINO-PURINE

By using the procedure described in Step K of Example 10 with (R,S)-E-(4-iodo-3-tetrahydropyranyloxy-1-butenyl)phosphonic acid diethyl ester (1.04 g, 2.5 mmol), the title product is obtained (0.415 g, 55%).

STEP E:

(R,S)-E-9-(4-DIHYDROXYPHOSPHORYL-2-HYDROXY-3-BUTENYL)GUANINE

By using the procedure described in Steps L and M of Example 10 with (R,S)-E-9-(4-diethoxyphosphoryl-2-tetrahydropyranyloxy-1-butenyl)-6-chloro-2-aminopurine (0.415 g, 0.9 mmol), the title product is obtained (0.16 g, 60%).

**EXAMPLE 23**

**(R,S)-E-9-(4-DIHYDROXYPHOSPHORYL-2-HYDROXY-3-BUTENYL)ADENINE**

STEP A:

(R,S)-E-9-(4-DIETHOXYPHOSPHORYL-2-TETRAHYDROPYRANYLOXY-3-BUTENYL)ADENINE

A mixture of (R,S)-E-(4-iodo-3-tetrahydropyranyloxy-1-butenyl)phosphonic acid diethyl ester (1.04 g, 2.5 mmol) in dimethylformamide (10 ml) is added to a mixture of adenine (0.35 g, 2.5 mmol) and sodium hydride (0.125 g, 50% in oil, 2.5 mmol) in dimethylformamide (10 ml) at 20°C under argon. Then, the mixture is stirred overnight at 20°C, concentrated *in vacuo*, diluted with brine and extracted with dichloromethane. The title product is obtained by flash chromatography on silica gel (0.6 g, 55%).

STEP B:

(R,S)-E-9-(4-DIETHOXYPHOSPHORYL-2-HYDROXY-3-BUTENYL)ADENINE

By using the procedure described in Step B of Example 11 with (R,S)-E-9-(4-diethoxyphosphoryl-2-tetrahydropyranyloxy-3-butenyl)adenine (0.6 g, 1.4 mmol), the title product is obtained (0.41 g, 85%).

STEP C:

(R,S)-E-9-(4-DIHYDROXYPHOSPHORYL-2-HYDROXY-3-BUTENYL)ADENINE

By using the procedure described in Step C of Example 11 with (R,S)-E-9-(4-diethoxyphosphoryl-2-hydroxy-3-butenyl)adenine (0.34 g, 1 mmol), the title product is obtained (0.2 g, 70%).

**EXAMPLE 24**

**(R,S)-E-1-(4-DIHYDROXYPHOSPHORYL-2-HYDROXY-3-BUTENYL)THYMINE**

STEP A:

(R,S)-E-1-(4-DIETHOXYPHOSPHORYL-2-TETRAHYDROPYRANYLOXY-3-BUTENYL)THYMINE

By using the procedure described in Step A of Example 2 with (R,S)-E-(4-iodo-3-tetrahydropyranyloxy-1-butenyl)phosphonic acid diethyl ester (1.04 g, 2.5 mmol) and thymine (0.315 g, 2.5 mmol), the title product is obtained (0.36 g, 35%).

STEP B:

(R,S)-E-(4-DIETHOXYPHOSPHORYL-2-HYDROXY-3-BUTENYL)THYMINE

By using the procedure described in Step B of Example 11 with (R,S)-E-1-(4-diethoxyphosphoryl-2-tetrahydropyranyloxy-3-butenyl)thymine (0.36 g, 0.87 mmol), the title product is obtained (0.235 g, 82%).

STEP C:

(R,S)-E-1-(4-DIHYDROXYPHOSPHORYL-2-HYDROXY-3-BUTENYL)THYMINE

By using the procedure described in Step B of Example 2 with (R,S)-E-(4-diethoxyphosphoryl-2-hydroxy-3-butenyl)thymine (0.22 g, 0.66 mmol), the title product is obtained (0.12 g, 65%).

**EXAMPLE 25**

**(R,S)-E-1-(4-DIHYDROXYPHOSPHORYL-2-HYDROXY-3-BUTENYL)CYTOSINE**

STEP A:

(R,S)-E-1-(4-DIETHOXYPHOSPHORYL-2-TETRAHYDROPYRANYLOXY-3-BUTENYL)-N-ACETYLCYTOSINE

By using the procedure described in Step E of Example 1 with (R,S)-E-(4-iodo-3-tetrahydropyranyloxy-1-butenyl)phosphonic acid diethyl ester (1.04 g, 2.5 mmol), the title product is obtained (0.83 g, 75%).

STEP B:

(R,S)-E-1-(4-DIETHOXYPHOSPHORYL-2-TETRAHYDROPYRANYLOXY-3-BUTENYL)CYTOSINE

By using the procedure described in Step F of Example 1 with (R,S)-E-1-(4-diethoxyphosphoryl-2-tetrahydropyranyloxy-3-butenyl)-N-acetylcytosine (0.8 g, 1.8 mmol), the title product is obtained (0.65 g, 85%).

STEP C:

(R,S)-E-1-(4-DIETHOXYPHOSPHORYL-2-HYDROXY-3-BUTENYL)CYTOSINE

By using the procedure described in Step B of Example 11 with (R,S)-E-1-(4-diethoxyphosphoryl-2-tetrahydropyranyloxy-3-butenyl)cytosine (0.64 g, 1.75 mmol), the title product is obtained (0.54 g, 90%).

STEP D:

(R,S)-E-1-(4-DIHYDROXYPHOSPHORYL-2-HYDROXY-3-BUTENYL)CYTOSINE

By using the procedure described in Step G of Example 1 with (R,S)-E-1-(4-diethoxyphosphoryl-2-hydroxy-3-butenyl)cytosine (0.51 g, 1.5 mmol), the title product is obtained (0.23 g, 55%).

**EXAMPLE 26**

**(R,S)-E-9-(5-DIHYDROXYPHOSPHORYL-3-HYDROXY-4-PENTENYL)GUANINE**

STEP A:

1-t.-BUTYLDIPHENYLSILYLOXY-3-BUTENE

By using the procedure described in Step A of Example 14 with 3-butene-1-ol (36 g, 500 mmol) the title product is obtained (124 g, 80%).

STEP B:

3-t.-BUTYLDIPHENYLSILYLOXYPROPANAL

By using the procedure described in Step B of Example 14 with 1-t.-butyldiphenylsilyloxy-3-butene (124 g, 400 mmol) the title product is obtained (87 g, 70%).

STEP C:

(R,S)-1-t.-BUTYLDIPHENYLSILYLOXY-3-HYDROXY-4-PENTENE

By using the procedure described in Step C of Example 14 with 3-t.-butyldiphenylsilyloxypropanal (87 g, 280 mmol) the title product is obtained (74 g, 78%).

STEP D:

(R,S)-1-t.-BUTYLDIPHENYLSILYLOXY-3-TETRAHYDROPYRANYLOXY-4-PENTENE

By using the procedure described in Step D of Example 14 with (R,S)-1-t.-butyldiphenylsilyloxy-3-hydroxy-4-pentene (68 g, 200 mmol) the title product is obtained (76 g, 90%).

STEP E:

(R,S)-2-TETRAHYDROPYRANYLOXY-4-t.-BUTYLDIPHENYLSILYLOXYBUTANAL

By using the procedure described in Step E of Example 14 with (R,S)-1-t.-butyldiphenylsilyloxy-3-tetrahydropyranyloxy-4-pentene (76 g, 180 mmol) the title product is obtained (56 g, 75%).

STEP F:

(R,S)-E-(5-t.BUTYLDIPHENYLSILYLOXY-3-TETRAHYDROPYRANYLOXY-1-PENTENYL)PHOSPHONIC ACID DIETHYL ESTER

By using the procedure described in Step H of Example 10 with (R,S)-2-tetrahydropyranyloxy-4-t.-butyldiphenylsilyloxybutanal (56 g, 135 mmol) the title product is obtained (56 g, 75%).

STEP G:

(R,S)-E-(5-MESYLOXY-3-TETRAHYDROPYRANYLOXY-1-PENTENYL)PHOSPHONIC ACID DIETHYL ESTER

By using the procedure described in Step C of Example 1 with (R,S)-E-(5-t.butyldiphenylsilyloxy-3-tetrahydropyranyloxy-1-pentenyl)phosphonic acid diethyl ester (56 g, 100 mmol) the title product is obtained (31 g, 75%).

STEP H:

(R,S)-E-(5-IODO-3-TETRAHYDROPYRANYLOXY-1-PENTENYL)PHOSPHONIC ACID DIETHYL ESTER

By using the procedure described in Step D of Example 1 with (R,S)-E-(5-mesyloxy-3-tetrahydropyranyloxy-1-pentenyl)phosphonic acid diethyl ester (31 g, 75 mmol) the title product is obtained (28 g, 85%).

STEP I:

(R,S)-E-9-(5-DIETHOXYPHOSPHORYL-3-TETRAHYDROPYRANYLOXY-4-PENTENYL)-6-CHLORO-2-AMINO-PURINE

By using the procedure described in Step K of Example 10 with (R,S)-E-(5-iodo-3-tetrahydropyranyloxy-1-pentenyl)phosphonic acid diethyl ester (7 g, 15.9 mmol) the title product is obtained (6.9 g, 75%).

STEP J:

(R,S)-E-9-(5-DIETHOXYPHOSPHORYL-3-HYDROXY-4-PENTENYL)GUANINE

By using the procedure described in Step L of Example 10 with (R,S)-E-9-(5-diethoxyphosphoryl-3-tetrahydropyranyloxy-4-pentenyl)-6-chloro-2-amino-purine (5.8 g, 10 mmol) the title product is obtained (4.05 g, 85%).

STEP K:

(R,S)-E-9-(5-DIHYDROXYPHOSPHORYL-3-HYDROXY-4-PENTENYL)GUANINE

By using the procedure described in Step M of Example 10 with (R,S)-E-9-(5-diethoxyphosphoryl-3-hydroxy-4-pentenyl)guanine (2.39 g, 5 mmol) the title product is obtained (1.37 g, 65%).

**EXAMPLE 27**

**(R,S)-E-9-(5-DIHYDROXYPHOSPHORYL-3-HYDROXY-4-PENTENYL)ADENINE**

STEP A:

(R,S)-E-9-(5-DIETHOXYPHOSPHORYL-3-TETRAHYDROPYRANYLOXY-4-PENTENYL)ADENINE

By using the procedure described in Step K of Example 10 with (R,S)-E-(5-iodo-3-tetrahydropyranyloxy-1-pentenyl)phosphonic acid diethyl ester (7 g, 15.9 mmol) and adenine (2.15 g, 16 mmol) the title product is obtained (5.36 g, 62%).

STEP B:

(R,S)-E-9-(5-DIETHOXYPHOSPHORYL-3-HYDROXY-4-PENTENYL)ADENINE

By using the procedure described in Step B of Example 11 with (R,S)-E-9-(5-diethoxyphosphoryl-3-tetrahydropyranyloxy-4-pentenyl)adenine (4.90 g, 9 mmol) the title product is obtained (3.6 g, 87%).

STEP C:

(R,S)-E-9-(5-DIHYDROXYPHOSPHORYL-3-HYDROXY-4-PENTENYL)ADENINE

By using the procedure described in Step C of Example 11 with (R,S)-E-9-(5-Diethoxyphosphoryl-3-hydroxy-4-pentenyl)adenine (3.2 g, 7 mmol) the title product is obtained (2.03 g, 72%).

**EXAMPLE 28**

**(R,S)-E-1-(5-DIHYDROXYPHOSPHORYL-3-HYDROXY-4-PENTENYL)THYMINE**

STEP A:

(R,S)-E-1-(5-DIETHOXYPHOSPHORYL-3-TETRAHYDROPYRANYLOXY-4-PENTENYL)THYMINE

By using the procedure described in Step A of Example 2 with (R,S)-E-(5-iodo-3-tetrahydropyranyloxy-1-pentenyl)phosphonic acid diethyl ester (7 g, 15.9 mmol) the title product is obtained (2.43 g, 35%).

STEP B:

(R,S)-E-1-(5-DIETHOXYPHOSPHORYL-3-HYDROXY-4-PENTENYL)THYMINE

By using the procedure described in Step B of Example 11 with (R,S)-E-1-(5-diethoxyphosphoryl-3-tetrahydropyranyloxy-4-pentenyl)thymine (2.14 g, 5 mmol) the title product is obtained (1.9 g, 85%).

STEP C:

(R,S)-E-1-(5-DIHYDROXYPHOSPHORYL-3-HYDROXY-4-PENTENYL)THYMINE

By using the procedure described in Step B of Example 2 with (R,S)-E-1-(5-diethoxyphosphoryl-3-hydroxy-4-pentenyl)thymine (1.9 g, 4.25 mmol) the title product is obtained (1.46 g, 87%).

**EXAMPLE 29**

**(R,S)-E-1-(5-DIHYDROXYPHOSPHORYL-3-HYDROXY-4-PENTENYL)CYTOSINE**

STEP A:

(R,S)-E-1-(5-DIETHOXYPHOSPHORYL-3-TETRAHYDROPYRANYLOXY-4-PENTENYL)-N-ACETYLCYTOSINE

By using the procedure described in Step D of Example 1 with (R,S)-E-(5-iodo-3-tetrahydropyranyloxy-1-pentenyl)phosphonic acid diethyl ester (7 g, 15.9 mmol) the title product is obtained (5.7 g, 77%).

STEP B:

(R,S)-E-(5-DIETHOXYPHOSPHORYL-3-TETRAHYDROPYRANYLOXY-4-PENTENYL)CYTOSINE

By using the procedure described in Step F of Example 1 with (R,S)-E-1-(5-diethoxyphosphoryl-3-tetrahydropyranyloxy-4-pentenyl)-N-acetylcytosine (5.6 g, 12 mmol) the title product is obtained (4.6 g, 92%).

STEP C:

(R,S)-E-(5-DIETHOXYPHOSPHORYL-3-HYDROXY-4-PENTENYL)CYTOSINE

By using the procedure described in Step B of Example 11 with (R,S)-E-(5-diethoxyphosphoryl-3-tetrahydropyranyloxy4-pentenyl)cytosine (4.2 g, 10 mmol) the title product is obtained (2.9 g, 85%).

STEP D:

(R,S)-E-1-(5-DIHYDROXYPHOSPHORYL-3-HYDROXY-4-PENTENYL)CYTOSINE

By using the procedure described in Step G of Example 1 with (R,S)-E-(5-diethoxyphosphoryl-3-hydroxy-4-pentenyl)cytosine (2.8 g, 8.25 mmol) the title product is obtained (1.6 g, 70%).

**EXAMPLE 30**

**(R,S)-E-9-(4-DIHYDROXYPHOSPHORYL-2-HYDROXYBUTYL-3-ENYLOXY)GUANINE**

STEP A:

(R,S)-E-9-(4-DIETHOXYPHOSPHORYL-2-TETRAHYDROPYRANYLOXY-BUT-3-ENYLOXY)-2-(DI-t.-BUTOXYCARBONYL)AMINO-6-METHOXYPURINE

To a mixture of 9-hydroxy-(di-t.-butoxycarbonyl)amino-6-methoxypurine (3.9 g, 10 mmol), (R,S)-E-(4-hydroxy-3-tetrahydropyranyloxy-1-butenyl)phosphonic acid diethyl ester, (3.05 g, 10 mmol), (described in Step A, Example 22) and triphenylphosphine (3.9 g, 15 mmol) stirred in anhydrous tetrahydrofuran (100 ml), diethylazodicarboxylate (2.6 g, 15 mmol) is added at 0°C under argon. Then, the mixture is warmed to 20°C and stirred for 2 hours. Then, the reaction mixture is concentrated *in vacuo* and the residue is purified by flash chromatography on silica gel to give the title product (3.7 g, 55%).

STEP B:

(R,S)-E-9-(4-DIHYDROXYPHOSPHORYL-2-HYDROXYBUT-3-ENYLOXY)GUANINE

By using the reaction sequence described in Steps L and M of Example 10 with (R,S)-E-9-(4-diethoxyphosphoryl-2-tetrahydropyranyloxy-but-3-enyloxy)-2-(di-t.-butoxycarbonyl)amino-6-methoxypurine (3.4 g, 5 mmol), the title product is obtained (0.88 g, 55%).

**EXAMPLE 31**

**(R,S)-E-9-(4-DIHYDROXYPHOSPHORYL-2-HYDROXYBUT-3-ENYLOXY)ADENINE**

STEP A:

(R,S)-E-9-(4-DIETHOXYPHOSPHORYL-2-TETRAHYDROPYRANYLOXYBUT-3-ENYLOXY)-6-PHTHALIMIDOPURINE

By using the procedure described in Step A of Example 30 with 9-hydroxy-6-phthalimidopurine (2.8 g, 10 mmol) the title product is obtained (3.7 g, 70%).

STEP B:

(R,S)-E-9-(4-DIETHOXYPHOSPHORYL-2-TETRAHYDROPYRANYLOXYBUT-3-ENYLOXY)ADENINE

A mixture of (R,S)-E-9-(4-diethoxyphosphoryl-2-tetrahydropyranyloxybut-3-enyloxy)-6-phthalimidopurine (2.85 g, 5 mmol) and methylhydrazine (0.23 g, 5 mmol) in anhydrous ethanol (15 ml) is stirred at 20°C overnight. Then, the reaction mixture is concentrated *in vacuo* and the title product is obtained by flash chromatography on silica gel (1.6 g, 75%).

STEP C:

(R,S)-E-9-(4-DIETHOXYPHOSPHORYL-2-HYDROXYBUT-3-ENYLOXY)ADENINE

By using the procedure described in Step B of Example 11 with (R,S)-E-9-(4-diethoxyphosphoryl-2-tetrahydropyranyloxybut-3-enyloxy)adenine (1.5 g, 3.4 mmol) the title product is obtained (1.03 g, 85%).

STEP D:

(R,S)-E-9-(4-DIHYDROXYPHOSPHORYL-2-HYDROXYBUT-3-ENYLOXY)ADENINE

By using the procedure described in Step C of Example 11 with (R,S)-E-9-(4-diethoxyphosphoryl-2-hydroxybut-3-enyloxy)adenine (0.71 g, 2 mmol) the title product is obtained (0.39 g, 65%).

**EXAMPLE 32**

**(R,S)-E-1-(4-DIHYDROXYPHOSPHORYL-2-HYDROXYBUT-3-ENYLOXY)THYMINE**

STEP A:

(R,S)-E-1-(4-DIETHOXYPHOSPHORYL-2-TETRAHYDROPYRANYLOXYBUT-3-ENYLOXY)THYMINE

By using the procedure described in Step A of Example 30 with 1-hydroxythymine (1.42 g, 10 mmol) the title product is obtained (3.10 g, 72%).

STEP B:

(R,S)-E-1-(4-DIETHOXYPHOSPHORYL-2-HYDROXYBUT-3-ENYLOXY)THYMINE

By using the procedure described in Step B of Example 21 with (R,S)-E-1-(4-diethoxyphosphoryl-2-tetrahydropyranyloxybut-3-enyloxy)thymine (3 g, 7 mmol) the title product is obtained (1.87 g, 77%).

STEP C:

(R,S)-E-1-(4-DIHYDROXYPHOSPHORYL-2-HYDROXYBUT-3-ENYLOXY)THYMINE

By using the procedure described in Step C of Example 21 with (R,S)-E-1-(4-diethoxyphosphoryl-2-hydroxybut-3-enyloxy)thymine (1.73 g, 5 mmol) the title product is obtained (0.91 g, 65%).

**EXAMPLE 33**

**(R,S)-E-1-(4-DIHYDROXYPHOSPHORYL-2-HYDROXYBUT-3-ENYLOXY)CYTOSINE**

STEP A:

(R,S)-E-1-(4-DIETHOXYPHOSPHORYL-2-TETRAHYDROPYRANYLOXYBUT-3-ENYLOXY)-N-BENZOYLCYTOSINE

By using the procedure described in Step A of Example 30 with 1-hydroxy-N-benzoylcytosine (2.3 g, 10 mmol) the title product is obtained (3.7 g, 71%).

STEP B:

(R,S)-E-1-(4-DIETHOXYPHOSPHORYL-2-HYDROXYBUTYL-3-ENYLOXY)CYTOSINE

By using the procedure described in Step B of Example 20 with (R,S)-E-1-(4-diethoxyphosphoryl-2-tetrahydropyranyloxybut-3- enyloxy)-N-benzoylcytosine (3.64 g, 7 mmol) the title product is obtained (1.9 g, 82%).

STEP C:

(R,S)-E-1-(4-DIHYDROXYPHOSPHORYL-2-HYDROXYBUT-3-ENYLOXY)CYTOSINE

By using the procedure described in Step C of Example 20 with (R,S)-E-1-(4-diethoxyphosphoryl-2-hydroxybut-3-enyloxy)cytosine (1.655 g, 5 mmol) the title product is obtained (0.78 g, 58%).

**EXAMPLE 34**

**[5-(6-AMINO-9H-PURIN-9-YL)-1-PENTENYL] PHOSPHONIC ACID**

STEP A:

5-(6-AMINO-9H-PURIN-9-YL)-1-PENTENE

27.72 g of cesium carbonate (85 mmol) are added to a stirred suspension of adenine (5 g, 37 mmol) in 80 ml of anhydrous dimethylformamide at 20°C under argon. After 30 minutes, 5-bromo-1-pentene (7.21 g, 48 mmol) is added to the reaction mixture which is stirred at 20°C for 20 hours, evaporated under reduced pressure and purified by flash chromatography on silica gel to give 5.34 g of the expected product 5-(6-amino-9H-purin-9-yl)-1-pentene (71% yield).

STEP B:

5-[(6-(Bisbenzoyl)amino-9H-purin-9-yl)]-1-pentene

Benzoyl chloride (14.05 g, 100 mmol) and 14 ml of triethylamine are successively added to a stirred solution of 8.6 g (42.5 mmol) of 5-(6-amino-9H-purin-9-yl)-1-pentene, dissolved in 100 ml of anhydrous dichloromethane at 20°C under argon. After 6 hours, 0.45 g of 4-dimethylaminopyridine are added to the reaction mixture which is stirred at 45°C for 15 hours. The crude mixture is washed with water, bicarbonate and brine, evaporated under reduced pressure and purified by flash chromatography on silica gel to give 14.8 g of the expected product 5-[(6-(bisbenzoyl)amino-9H-purin-9-yl)]-1-pentene (85% yield).

STEP C:

[5-[(6-(BISBENZOYL)AMINO-9H-PURIN-9-YL)]-1-PENTENYL]PHOSPHONIC ACID, DIETHYL ESTER

Ozone (10 mmol) is bubbled through a solution of 5-[(6-(bisbenzoyl)amino-9H-purin-9-yl)]-1-pentene (2.52 g, 6.13 mmol) in 6 ml of dichloromethane and 3 ml of methanol, at -78°C. After 15 minutes, excess ozone is removed by bubbling nitrogen to the solution at -78°C, and 6.5 mmol of dimethyl sulfide are added to the reaction mixture which is slowly stirred to 20°C. The crude mixture is evaporated under reduced pressure and purified by flash chromatography on silica gel to give 1.47 g of aldehyde (66%) which is suspended in 5 ml of tetrahydrofuran and added to a stirred solution of the lithium salt of tetraethyl methylene bisphosphonate (prepared by adding 5.3 mmol of n-butyllithium to 5.3 mmol of tetraethylmethylene bisphosphonate on 8 ml of tetrahydrofuran at -78°C under argon). The reaction mixture is stirred at -78°C for 3 hours, at 20°C for 5 hours, quenched with saturated aqueous ammonium chloride and evaporated. The residue is extracted with ethyl acetate (5 x 30 ml). The organic layers are evaporated and purified by flash chromatography on silica gel to give 1.37 g of the expected product [5-[(6-(bisbenzoyl)amino-9H-purin-9-yl)]-1-pentenyl] phosphonic acid, diethyl ester.

STEP D:

[5-(6-AMINO-9H-PURIN-9-YL)-1-PENTENYL] PHOSPHONIC ACID

Sodium methoxide (0.1 g) is added to a stirred solution of 0.92 g (1.68 mmol) of [5-[(6-(bisbenzoyl)amino-9H-purin-9-yl)]-1-pentenyl phosphonic acid, diethyl ester dissolved in 5 ml of methanol at 20°C. After stirring for 20 hours, the reaction mixture is evaporated and purified by flash chromatography on silica gel to give 0.47 g (83%) of [5-(6-amino-9H-purin-9-yl)-1-pentenyl] phosphonic acid, diethyl ester. This compound is dissolved in 5 ml of anhydrous acetonitrile and heated by 0.7 ml (5.4 mmol) of trimethylsilylbromide at 20°C under argon. After stirring for 18 hours at 20°C, the reaction mixture is evaporated under reduced pressure. The residue is dissolved in acetonitrile and precipitates upon addition of water. Recrystallization from ethanol gives 0.65 g of the title compound [5-(6-amino-9H-purin-9-yl)-1-pentenyl] phosphonic acid.

The compounds of this invention are useful in the medical therapy particularly for the treatment or prophylaxis of viral infections such as for example antiviral agents effective against DNA viruses (herpes viruses 1 and 2, cytomegalovirus, varicella-zoster virus, Epstein-Barr virus), retroviruses (human immunodeficiency viruses 1 and 2 and visna virus) and related clinical conditions such as AIDS-related complex (ARC) and against viruses involved in tumor formation. The antiviral agents of the present invention have usefulness as monotherapy agents and in conjunction with other antiviral agents such as in conjunctive therapy for the treatment of retroviral infections, especially in humans, particularly human immunodeficiency virus. Particularly preferred is conjunctive therapy with 2′,3′-dideoxy purine nucleosides are 2′3′-dideoxyadenosine, 2′,3′-dideoxyguanosine, 2′,3′-dideoxythioinosine and 2′,3′-dideoxyinosine. Other possible conjunctive therapy include agents that are effective for the treatment or prophylaxis of viral infections or associated conditions such as 3′-azido-3′-deoxythymidine (zidovudine), 2′,3′-dideoxynucleosides such as 2′,3′-dideoxycytidine, 2′,3′-dideoxy adenosine and 2′,3′-dideoxyinosine, acyclic nucleosides (e.g. acyclovir), interferons such as α-interferon, renal excretion inhibitors such as probenicid, nucleoside transport inhibitors such as dipyridamole, as well as immunomodulators such as interleukin II and granulocyte macrophage colony stimulating factors. The component compounds of such combination therapy may be administered simultaneously, in either separate or combined formulations, or at different times, e.g., sequentially such that a combined effect is achieved.

The antiviral efficacy of the compounds of the present invention may be determined by any appropriate method. Some representative methods of testing the efficacy of these compounds follow.

## MTT cell viability assay for Human Immunodeficiency virus (HIV)

The MTT cell viability assay was originally described by Pauwels et al., (J. Virol. Methods, 1988: 20, 309-321). It is a colorimetric assay based on the ability of viable but not dead cells to reduce yellow colored 3-(4,5-dimethyl-2-yl)-2,5-diphenyl-tetrazolium bromide (MTT) (Sigma Chemical Co. Ltd.) to a blue formazan product. This reduction reaction is carried out by mitochondrial dehydrogenases of metabolically active cells. The assay permits a rapid and accurate estimate of the anti-HIV activity of potential antiviral agents in parallel with their cytotoxicity enabling selectivity indices (S.I) to be determined.

MT-4 cells which are highly susceptible to HIV infection are used infected with the HIV-1 strain RF. The central 60 wells of plastic 96 well flat-bottomed microtitre trays (Sterilin Ltd.) are filled with 100 μl of growth medium containing serial dilutions of the test compounds at twice the required final concentration. The outside wells are filled with sterile distilled water to prevent evaporation during the incubation period. For each concentration of compound, there are two sets of triplicate wells, so that the effect of the compound on both infected and uninfected cells can be evaluated simultaneously. Some wells are left drug free as untreated controls for both mock- and virus-infected cells. Exponentially growing MT-4 cells are counted and the number of cells adjusted to allow 5 x $10^4$ cells per well. The cells are then pelleted and divided into two. Half of the cells are infected with virus (100TCID50 per 5 x $10^4$ cells) and the other half mock infected. Virus adsorption is for one hour at room temperature. The cells are then pelleted and washed once in RPMI prior to being resuspended in a volume of medium such that 100 μl can be added to each well of the microtitre plate. The culture plates are incubated at 37°C in an incubator containing 5% $CO_2$.

After six days incubation, 10 μl of a solution of MTT (7.5 mg/ml) in PBS is added to each well and the plates incubated for a further one hour at 37°C. The formazan crystals are solubilized by adding 100 μl of 10% (v/v) Triton X-100 in acidified isopropanol (2 ml concentrated HCl per 500 ml solvent) and mixing. Finally the absorbance is read at 540 nm using a Multiskan MCC spectrophotometer (Flow Laboratories). For each compound, the mean optical density (O.D.) readings for both mock- and virus-infected cells are plotted against the drug concentration. The O.D. value representing the 50% endpoint from which both the 50% cytotoxic dose (CD50) and 50% inhibitory concentration (IC50) of the test compounds can be determined is calculated using the following formula:

$$\frac{\text{mean O.D. mock infected} - \text{mean O.D. virus infected}}{2}$$

## Protocol for Detection of anti-HIV activity of compounds using C-8166

The central 60 wells of plastic 96 well flat-bottomed microtitre trays are filled with 100 μl of growth medium containing serial dilutions of the test compounds at twice the required final concentration. The outside wells are fitted with sterile distilled water to prevent evaporation during the incubation period. Triplicate wells are used for each concentration of compound. Some wells are left drug free as untreated controls. Exponentially growing C-8166 cells are counted and the number of cells adjusted to allow 1 x $10^5$ cells per well. The cells are pelleted and infected with HIV to give a multiplicity of infection of between 0.001 and 0.0001 infectious units per cell.

Virus adsorption is for one hour at room temperature. The cells are then pelleted and washed three times in RPMI prior to being resuspended in a volume of medium such that 100 μl can be added to each well of the microtitre plates. The culture plates are incubated at 37°C in an incubator containing 5% $CO_2$. After three days the infected cells are observed and scored for the presence of syncytia: +++ =50%-100% cpe; ++ = 10%-50% cpe; + = <10% cpe and O = no syncytia. 100 μl of supernatant fluid is then harvested from each well and assayed for levels of p24 viral core antigen using an ELISA.

**p24 ELISA**

The central 60 wells of 96 well 'U' bottomed microtitre plates (Falcon, Becton Dickinson) are coated with 100 μl of an affinity purified sheep anti-H1V-1-p24 (Aalto Bio-reagents, Rathfarnham, Dublin, Ireland, code D7320) at a concentration of 10 μg/ml in coating buffer (100 mM $NaHCO_3$ pH 8.5). This product was produced by immunizing sheep with three different synthetic peptides corresponding to amino acids 283-297 (LDIRQGPKEPFRDYV); 173-188 (SALSEGATPQDLNTML) and 226-237 (GQMREPRGSDIA) of the p24 *gag* protein of HIV-1 strain BH-10. The plates are left for the antibodies to attach at +4°C overnight and then washed twice in Tris buffered saline (TBS) (0.144M NaCl, 25 mM Tris pH 7.5) using a microtitre plate washer (Luminar Technologies) prior to being blocked with 2% skimmed milk (Cadburys Marvel) made up in TBS for 1 hour at room temperature (200 μl/well). After two washes with TBS, 100 μl volumes of cell free culture fluid are added to the wells along with 10 μl of a 1% (v/v) solution of the zwitterionic detergent Empigen (Calbiochem). Depending on the expected levels of p24, the culture fluids are screened either neat or at 1:10 or 1:100 dilutions. The samples are incubated at room temperature overnight prior to the wells being washed three times and incubated with 100 μl of a second anti-p24 antibody, EH12E1-AP directly conjugated to alkaline phosphatase (ADP 452) at a concentration of 1:3000 in TBS containing 20% sheep sera (Seralab), 2% skimmed milk (Cadburys Marvel) and 0.5% Tween 20 (Sigma Chemical Co.). This antibody raised against the HIV-1 CBI-1 isolatedby Bridget Ferns, Richard Tedder and colleagues at the Middlessex Hospital Medical School has been mapped to a complex epitope incorporating two distinct peptide sequences. These are GHQAAMQMLKETINEEAAEWDRVHPVHAGPIAPGQ (aa 193-227) and NPPIPVGEIYKRWII (aa 253-267) and are conserved between HIV-1 strains. The alkaline phosphatase conjugate of EH12E1 (EH12E1-AP) was prepared by Novo BioLabs, Cambridge, U.K. This conjugated antibody was obtained through the ADP reagent bank. The plates are incubated at 37°C for 1 hour using an incubator/shaker (Luminar Technologies) and then the wells washed three times in TBS. The wells are given a final wash in buffer provided in the commercially available alkaline phosphatase detection and amplification kits, AMPAK (IQ (Bio) Ltd.) and 50 μl of the AMPAK substrate added according to the manufacturer's instructions. After 30 minutes at room temperature, 50 μl of the AMPAK amplifier is added and the purple color intensity read after stopping the reaction with acid at 492 nm using a Multiskan MCC/340 Spectrophotometer (Flow Laboratories) after approximately ten minutes.

The immunoassay is calibrated using recombinant HIV-1p24 (American Biotechnologies Inc.) obtained through the ADP, using a series of doubling dilutions starting at 100 ng/ml. This assay usually gives a linear response over the range 300 to 10,000 pg/ml, although there is day to day variation.

**Detection of the anti-Herpes (HSV-1 and HSV-2 activity of compounds**

For assay, HeLa (human cervical carcinoma) cells ($0.8 \times 10^5$/0.1 ml) or Vero (African Green Monkey kidney) cells ($1.0 \times 10^5$/0.1 ml) in the appropriate growth medium were transferred to flat-bottomed, 96 well (0.1 ml cells/well) microtitre plates (Falcon). After 24 hours' incubation at 37°C in a humidified $CO_2$ (5% $CO_2$, 95% air) incubator the cultures were ready for use.

For each assay the growth medium was aspirated from the microtitre plate cultures and replaced with 100 μl maintenance medium (cell and virus controls) or compound diluted to twice test concentration in maintenance medium (toxicity controls, test wells). After 3 hours' incubation at 37°C in a humidified $CO_2$ incubator each culture received 100 μl maintenance medium (cell and toxicity controls) or virus [Herpes simplex virus type 1 (HSV-1; strain HF, ATCC VR-260) or Herpes simplex virus type 2 (HSV-2; strain G, ATCC VR-734)] diluted in maintenance medium (virus controls, compound test wells). All cultures were then incubated at 37°C and examined microscopically at 48 and 72 hours (Herpes) or 7, 10 and 14 days (CMV) for virus- and compound-induced cytopathic effect (CPE). CPE was graded as 0 (no), 1+ (25%), 3+ (75%) or 4+ (100%) cell monolayer destruction. These data were then used to calculate the 50% compound inhibitory concentrations ($IC_{50}$'s).

**Detection of the anti-cytomegalovirus activity of compounds**

For assay, MRC-5 cells ($1.2 \times 10^5$/0.1 ml) in the appropriate growth medium were transferred to flat-bottomed, 96 well (0.1 ml cells/well) microtitre plates (Falcon). After 24 hours' incubation at 37°C in a humidified $CO_2$ (5% $CO_2$, 95% air) incubator the cultures were ready for use.

For each assay the growth medium was aspirated from the microtitre plate cultures and replaced with 100 μl maintenance medium (cell and virus controls) or compound diluted to twice test concentration in maintenance medium (toxicity controls, test wells). After 3 hours' incubation at 37°C in a humidified $CO_2$ incubator each culture received 100 μl maintenance medium (cell and toxicity controls) or virus [Human Cytomegalovirus (CMV, strain AD-169, ATCC VR-538)] diluted in maintenance medium (virus controls, compound test wells). All cultures were then incubated at 37°C and examined microscopically at 48 and 72 hours (Herpes) or 7, 10 and 14 days (CMV) for virus- and compound-induced cytopathic effect (CPE). CPE was graded as 0 (no), 1+ (25%), 3+ (75%) or 4+ (100%) cell monolayer destruction. These data were then used to calculate the 50% compound inhibitory concentrations ($IC_{50}$'s).

## DETERMINATION OF THE EFFICIENCY OF PHOSPHORYLATION OF THE ACYCLONUCLEOTIDE DERIVATIVES OF GUANINE BY GUANYLATE KINASE

The efficiency of phosphorylation (defined as the Vmax/Km ratio) of the acyclonucleotide derivatives of guanine by guanylate kinase was compared to that of GMP, used as reference substrate. The assay used for determination of the parameters Vmax and Km was as described by Navé et al. in Arch. Biochem. Biophys. (1992), 295, 253-257.

The amount of the active ingredient to be administered can vary widely according to the particular dosage unit employed, the period of treatment, the age and sex of the patient treated and the nature and extent of the disorder treated. The total amount effective antiviral amount of the active ingredient to be administered will generally range from about 1 mg/kg to 100 mg/kg and preferably from 3 mg/kg to 25 mg/kg. A unit dosage may contain from 25 to 500 mg of active ingredient, and can be taken one or more times per day. The active compound of formula I or II can be administered with a pharmaceutical carrier using conventional dosage unit forms either orally, parenterally, topically or transdermally.

As used herein the term "patient" includes mammals such as mice, rats, cats, dogs, cattle, sheep, swine, and primates including humans.

For oral administration the compounds can be formulated into solid or liquid preparations such as capsules, pills, tablets, troches, lozenges, melts, powders, solutions, suspensions, or emulsions. The solid unit dosage forms can be a capsule which can be of the ordinary hard- or soft-shelled gelatin type containing, for example, surfactants, lubricants, and inert fillers such as lactose, sucrose, calcium phosphate, and cornstarch. In another embodiment the compounds of this invention can be tableted with conventional tablet bases such as lactose, sucrose, and cornstarch in combination with binders such as acacia, cornstarch, or gelatin, disintegrating agents intended to assist the break-up and dissolution of the tablet following administration such as potato starch, alginic acid, corn starch, and guar gum, lubricants intended to improve the flow of tablet granulations and to prevent the adhesion of tablet material to the surfaces of the tablet dies and punches, for example, talc, stearic acid, or magnesium, calcium, or zinc stearate, dyes, coloring agents, and flavoring agents intended to enhance the aesthetic qualities of the tablets and make them more acceptable to the patient. Suitable excipients for use in oral liquid dosage forms include diluents such as water and alcohols, for example, ethanol, benzyl alcohol, and the polyethylene alcohols, either with or without the addition of a pharmaceutically acceptable surfactant, suspending agent, or emulsifying agent.

The compounds of this invention may also be administered parenterally, that is, subcutaneously, intravenously, intramuscularly, or interperitoneally, as injectable dosages of the compound in a physiologically acceptable diluent with a pharmaceutical carrier which can be a sterile liquid or mixture of liquids such as water, saline, aqueous dextrose and related sugar solutions, an alcohol such as ethanol, isopropanol, or hexadecyl alcohol, glycols such as propylene glycol or polyethylene glycol, glycerol ketals such as 2,2-dimethyl-1,3-dioxolane-4-methanol, ethers such as poly(ethyleneglycol) 400, an oil, a fatty acid, a fatty acid ester or glyceride, or an acetylated fatty acid glyceride with or without the addition of a pharmaceutically acceptable surfactant such as a soap or a detergent, suspending agent such as pectin, carbomers, methylcellulose, hydroxypropylmethylcellulose, or carboxymethylcellulose, or emulsifying agent and other pharmaceutical adjuvants. Illustrative of oils which can be used in the parenteral formulations of this invention are those of petroleum, animal, vegetable, or synthetic origin, for example, peanut oil, soybean oil, sesame oil, cottonseed oil, corn oil, olive oil, petrolatum, and mineral oil. Suitable fatty acids include oleic acid, stearic acid, and isostearic acid. Suitable fatty acid esters are, for example, ethyl oleate and isopropyl myristate. Suitable soaps include fatty alkali metal, ammonium, and triethanolamine salts and suitable detergents include cationic detergents, for example, dimethyl dialkyl ammonium halides, alkyl pyridinium halides, and alkylamines acetates; anionic detergents, for example, alkyl, aryl, and olefin sulfonates, alkyl, olefin, ether, and monoglyceride sulfates, and sulfosuccinates; nonionic detergents, for example, fatty amine oxides, fatty acid alkanolamides, and polyoxyethylenepolypropylene copolymers; and amphoteric detergents, for example, alkyl-beta-aminopropionates, and 2-alkylimidazoline quarternary ammonium salts, as well as mixtures. The parenteral compositions of this invention will typically contain from about 0.5 to about 25% by weight of the active ingredient in solution. Preservatives and buffers may also be used advantageously. In order to minimize or eliminate irritation at the site of injection, such compositions may contain a non-ionic surfactant having a hydrophile-lipophile balance (HLB) of from about 12 to about 17. The quantity of surfactant in such formulations ranges from about 5 to about 15% by

weight. The surfactant can be a single component having the above HLB or can be a mixture of two or more components having the desired HLB. Illustrative of surfactants used in parenteral formulations are the class of polyethylene sorbitan fatty acid esters, for example, sorbitan monooleate and the high molecular weight adducts of ethylene oxide with a hydrophobic base, formed by the condensation of propylene oxide with propylene glycol.

The compounds of this invention can also be administered topically. This can be accomplished by simply preparing a solution of the compound to be administered, preferably using a solvent known to promote transdermal absorption such as ethanol or dimethyl sulfoxide (DMSO) with or without other excipients. Preferably topical administration will be accomplished using a patch either of the reservoir and porous membrane type or of a solid matrix variety.

Some suitable transdermal devices are described in U.S. Pat. Nos. 3,742,951, 3,797,494, 3,996,934, and 4,031,894. These devices generally contain a backing member which defines one of its face surfaces, an active agent permeable adhesive layer defining the other face surface and at least one reservoir containing the active agent interposed between the face surfaces. Alternatively, the active agent may be contained in a plurality of microcapsules distributed throughout the permeable adhesive layer. In either case, the active agent is delivered continuously from the reservoir or microcapsules through a membrane into the active agent permeable adhesive, which is in contact with the skin or mucosa of the recipient. If the active agent is absorbed through the skin, a controlled and predetermined flow of the active agent is administered to the recipient. In the case of microcapsules, the encapsulating agent may also function as the membrane.

In another device for transdermally administering the compounds in accordance with the present invention, the pharmaceutically active compound is contained in a matrix from which it is delivered in the desired gradual, constant and controlled rate. The matrix is permeable to the release of the compound through diffusion or microporous flow. The release is rate controlling. Such a system, which requires no membrane is described in U.S. Pat. No. 3,921,636. At least two types of release are possible in these systems. Release by diffusion occurs when the matrix is non-porous. The pharmaceutically effective compound dissolves in and diffuses through the matrix itself. Release by microporous flow occurs when the pharmaceutically effective compound is transported through a liquid phase in the pores of the matrix.

## Claims

1. A compound of the formula:

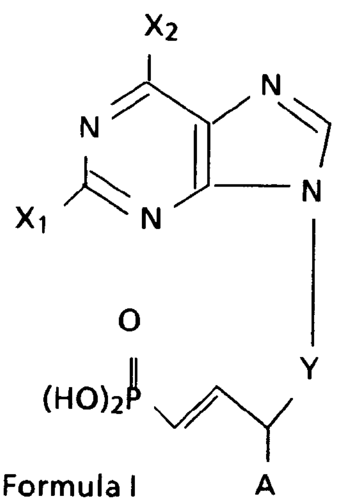

Formula I

or

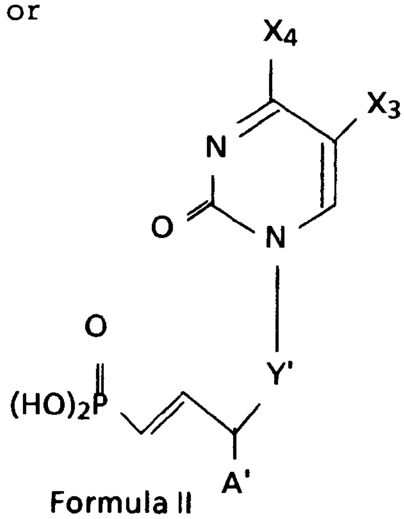

Formula II

the stereoisomeric forms and mixtures thereof, tautomeric forms and the pharmaceutically acceptable salts thereof, wherein

Y is $-CH_2-CH_2-$, $-O-CH_2-$ and $-CH_2-O-$;
Y′ is $(CH_2)_n$, $-O-CH_2-$, or $-CH_2-O-$;
n is 1 or 2;
A and A′ are independently H, $-CH_2OH$ or OH;

$X_1$ is H or $NH_2$;
$X_2$ is OH or $NH_2$;
$X_3$ is H or $CH_3$; and
$X_4$ is $NH_2$ or OH;
provided that when Y is -O-$CH_2$- and A is H, then $X_1$ is not $NH_2$ and $X_2$ is not OH, simultaneously;
provided that when Y -$CH_2$-O-, then A is OH;
provided that when A is OH, Y is not -$OCH_2$-; when A′ is OH, Y′ is not -$OCH_2$-; and
provided that when Y is -$CH_2$-$CH_2$- and A is H, then $X_1$ is not $NH_2$ and $X_2$ is not OH, simultaneously.

2. The compounds of Claim 1 wherein the compound is only from formula I.

3. The compounds of Claim 1 wherein the compound is only from formula II.

4. The compounds of Claim 1 wherein the compound is
E-1-[5-(dihydroxyphosphoryl)pent-4-enyl]cytosine;
E-1-[5-(dihydroxyphosphoryl)pent-4-enyl]thymine;
E-1-[4-(dihydroxyphosphoryl)but-3-enyl]cytosine;
E-1-[4-(dihydroxyphosphoryl)but-3-enyl]thymine;
E-1-[(3 -dihydroxyphosphoryl-prop-2-enyloxy)methyl]cytosine;
E-1-[(3-dihydroxyphosphoryl-2-propenyloxy)methyl]thymine;
E-9-[(3-dihydroxyphosphoryl-2-propenyloxy)methyl]adenine;
E-1-[4-(dihydroxyphosphoryl)but-3-enyloxy]cytosine;
E-1-[4-(dihydroxyphosphoryl)but-3-enyloxy]thymine;
E-9-[5-(dihydroxyphosphoryl)-3-hydroxymethyl-4-pentenyl]guanine;
(R,S)-E-9-[5-(dihydroxyphosphoryl)-3-(hydroxymethyl)-4-pentenyl]adenine;
(R,S)-E-1-(5-dihydroxyphosphoryl-3-hydroxymethyl-4-pentenyl)cytosine;
(R,S)-E-1-(5-dihydroxyphosphoryl-3-hydroxymethyl-4-pentenyl)thymine;
(R,S)-E-9-[(3-dihydroxyphosphoryl-1-hydroxymethyl-2-propenyloxy)methyl]guanine;
(R,S)-E-9-[(3-dihydroxyphosphoryl-1-hydroxymethyl-2-propenyloxy)methyl]adenine;
(R,S)-E-1-[(3-dihydroxyphosphoryl-1-hydroxymethyl-2-propenyloxy)methyl]cytosine;
(R,S)-E-1-[(3-dihydroxyphosphoryl-1-hydroxymethyl-2-propenyloxy)methyl]thymine;
(R,S)-E-1-[4-(dihydroxyphosphoryl)-2-(hydroxymethyl)-3-butenyl]cytosine;
E-1-[4-(dihydroxyphosphoryl)-2-hydroxymethyl-3-butenyl]thymine;
(R,S)-E-1-[4-(dihydroxyphosphoryl)-2-hydroxymethyl-but-3-enyloxy]cytosine;
(R,S)-E-1-[4-(dihydroxyphosphoryl)-2-hydroxymethyl-but-3-enyloxy]thymine;
(R,S)-E-9-(4-dihydroxyphosphoryl-2-hydroxy-3-butenyl)guanine;
(R,S)-E-9-(4-dihydroxyphosphoryl-2-hydroxy-3-butenyl)adenine;
(R,S)-E-1-(4-dihydroxyphosphoryl-2-hydroxy-3-butenyl)thymine;
(R,S)-E-1-(4-dihydroxyphosphoryl-2-hydroxy-3-butenyl)cytosine;
(R,S)-E-9-(5-dihydroxyphosphoryl-3-hydroxy-4-pentenyl)guanine;
(R,S)-E-9-(5-dihydroxyphosphoryl-3-hydroxy-4-pentenyl)adenine;
(R,S)-E-1-(5-dihydroxyphosphoryl-3-hydroxy-4-pentenyl)thymine;
(R,S)-E-1-(5-dihydroxyphosphoryl-3-hydroxy-4-pentenyl)cytosine;
(R,S)-E-9-(4-dihydroxyphosphoryl-2-hydroxybutyl-3-enyloxy)-guanine;
(R,S)-E-9-(4-dihydroxyphosphoryl-2-hydroxybut-3-enyloxy)-adenine;
(R,S)-E-1-(4-dihydroxyphosphoryl-2-hydroxybut-3-enyloxy)-thymine;
(R,S)-E-1-(4-dihydroxyphosphoryl-2-hydroxybut-3-enyloxy)cytosine; or
[5-(6-amino-9H-purin-9-yl)-1-pentenyl] phosphonic acid.

5. The compounds of claim 1 wherein $X_1$ is H and $X_2$ is $NH_2$.

6. The compounds of claim 1 wherein $X_1$ is $NH_2$ and $X_2$ is $NH_2$.

7. The compounds of claim 1 wherein $X_1$ is $NH_2$ and $X_2$ is OH.

8. The compounds of claim 1 wherein $X_1$ is H and $X_2$ is OH.

9. The compounds of claim 1 wherein $X_4$ is $NH_2$ and $X_3$ is H.

**10.** The compounds of claim 1 wherein $X_4$ is OH and $X_3$ is $CH_3$.

**11.** The compounds of claim 1 wherein Y is $-CH_2CH_2-$ or Y' is $(CH_2)_n$.

**12.** A compound according to any one of claims 1 to 11 for use as a pharmaceutically active compound.

**13.** A pharmaceutical composition containing a compound according to any one of claims 1 to 11, optionally in combination with a pharmaceutically acceptable carrier.

**14.** The pharmaceutical composition according to claim 13 useful for the treatment of diseases caused by DNA viruses, retroviruses or viruses involved in tumor formation.

**15.** Use of a compound according to any one of claims 1 to 11 optionally in combination with a pharmaceutically acceptable carrier, for the preparation of a pharmaceutical composition useful for the treatment of diseases caused by DNA viruses, retroviruses or viruses involved in tumor formation.

**16.** A method of preparing the compounds of the formulae :

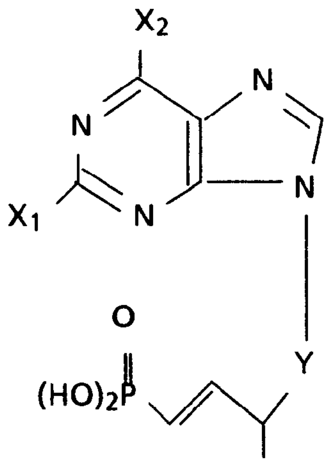

Formula I

or

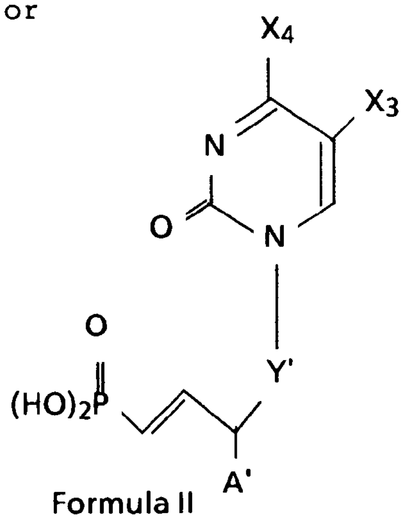

Formula II

the stereoisomeric forms and mixtures thereof, tautomeric forms and the pharmaceutically acceptable salts thereof, wherein

Y is $-CH_2-CH_2-$, $-O-CH_2-$ and $-CH_2-O-$;
Y' is $(CH_2)_n$, $-O-CH_2-$, or $-CH_2-O-$;
n is 1 or 2;
A and A' are independently H, $-CH_2OH$ or OH;
$X_1$ is H or $NH_2$;
$X_2$ is OH or $NH_2$;
$X_3$ is H or $CH_3$; and
$X_4$ is $NH_2$ or OH;

provided that when Y is $-O-CH_2-$ and A is H, then $X_1$ is not $NH_2$ and $X_2$ is not OH, simultaneously;
provided that when Y $-CH_2-O-$, then A is OH;
provided that when A is OH, Y is not $-OCH_2-$; when A' is OH, Y' is not $-OCH_2-$; and
provided that when Y is $-CH_2-CH_2-$ and A is H, then $X_1$ is not $NH_2$ and $X_2$ is not OH, simultaneously;
wherein the formulas (6), (7), or (8)

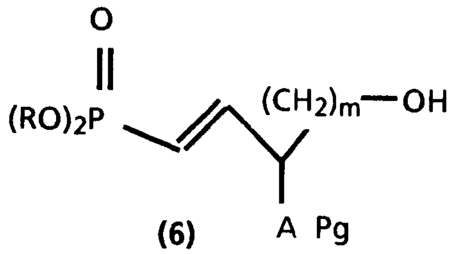

(6)

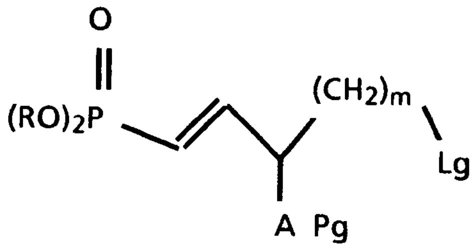

(7)

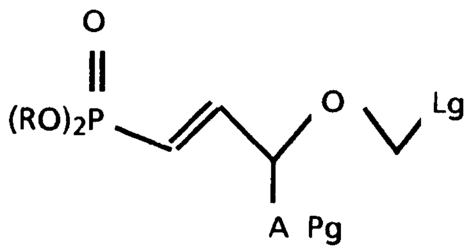

(8)

wherein m is 0, 1, or 2, R is $C_{1-8}$ alkyl, A is as defined in claim 1, and Pg is an appropriate protecting group when A is OH or $CH_2OH$, and Lg is an appropriate leaving group; are respectively reacted with

a 1-hydroxy pyrimidine derivative or 9-hydroxy purine derivative and an appropriate condensing agent in the presence of an organic solvent,

a salt form of the pyrimidine derivative or purine derivative in the presence of an organic solvent, and

a silylated derivative of the pyrimidine derivative or purine derivative in the presence of an organic solvent in an inert atmosphere, wherein the pyrimidine or purine derivative has the following formulae as defined previously, wherein the dotted line represents the point of attachment:

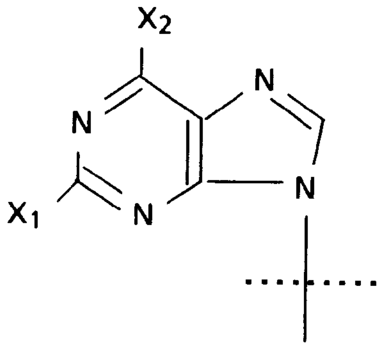

purine derivative

or

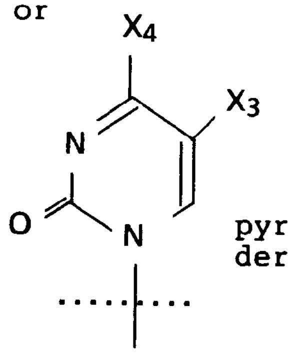

pyrimidine derivative

and optionally deprotecting the compound so produced; and recovering the desired product from the reaction zone.

European Patent Office

# EUROPEAN SEARCH REPORT

Application Number

EP 92 40 2487

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | NUCLEOSIDES & NUCLEOTIDES (PROCEEDINGS OF THE 9TH INTERNATIONAL ROUND TABLE, Uppsala, 30th July - 3rd August 1990), vol. 10, nos. 1-3, 1991, pages 275-278, Marcel Dekker, Inc.; S. PHADTARE et al.: "Unsaturated analogues of acyclic nucleoside phosphonates: An unusual arbuzov reaction with unactivated triple bond"<br>* Whole document *<br>--- | 1 | C 07 F 9/6561<br>A 61 K 31/675<br>C 07 F 9/6512 |
| A | COLLECTION OF CZECHOSLOVAK CHEMICAL COMMUNUMICATIONS, vol. 53, no. 11, November 1988, pages 2753-2777; I. ROSENBERG et al.: "Phosphonylmethoxyalkyl and phosphonylalkyl derivatives of adenine"<br>* Whole document *<br>--- | 1 | |
| P,A | WO-A-9 201 698 (BEECHAM GROUP PLC)<br>* Whole document *<br>----- | 1,2,4-8,12-16 | TECHNICAL FIELDS SEARCHED (Int. Cl.5)<br>C 07 F 9/00<br>A 61 K 31/00 |

The present search report has been drawn up for all claims

| Place of search | Date of completion of the search | Examiner |
|---|---|---|
| THE HAGUE | 26-11-1992 | BESLIER L.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)